# EUROPÄISCHE PATENTSCHRIFT

(11) **EP 1 140 977 B1**
(45) Veröffentlichungstag und Bekanntmachung des Hinweises auf die Patenterteilung: **09.03.2005**
(21) Anmeldenummer: 99965395.9
(22) Anmeldetag: 14.12.1999
(51) Int. Cl.: C07K 1/04, C07H 21/00, B01J 19/00

(54) **VERFAHREN UND VORRICHTUNG ZUM AUFBRINGEN VON SUBSTANZEN AUF EINEN TRÄGER, INSBESONDERE VON MONOMEREN FÜR DIE KOMBINATORISCHE SYNTHESE VON MOLEKÜLBIBLIOTHEKEN**
METHOD AND DEVICES FOR APPLYING SUBSTANCES TO A SUPPORT, ESPECIALLY MONOMERS FOR THE COMBINATORIAL SYNTHESIS OF MOLECULE LIBRARIES
PROCEDES ET DISPOSITIFS PERMETTANT D'APPLIQUER DES SUBSTANCES SUR UN SUBSTRAT, NOTAMMENT DE MONOMERES POUR LA SYNTHESE COMBINATOIRE DE BIBLIOTHEQUES DE MOLECULES

(30) Priorität: 14.12.1998 DE 19857529; 30.07.1999 DE 19935553
(43) Veröffentlichungstag der Anmeldung: 10.10.2001
(73) Patentinhaber: Deutsches Krebsforschungszentrum Stiftung des öffentlichen Rechts, 69120 Heidelberg (DE); Europäisches Laboratorium für Molekularbiologie, D-69117 Heidelberg (DE)
(72) Erfinder: POUSTKA, Annemarie, D-69120 Heidelberg (DE); BREITLING, Frank, D-69115 Heidelberg (DE); GROSS, Karl-Heinz, D-69221 Dossenheim (DE); DÜBEL, Stefan, D-69221 Dossenheim (DE); SAFFRICH, Rainer, D-69221 Dossenheim (DE)
(74) Vertreter: Castell, Klaus, Dr.-Ing.
(86) Internationale Anmeldenummer: PCT/DE1999/003982
(87) Internationale Veröffentlichungsnummer: WO 2000/035940

(56) Entgegenhaltungen:
- WO-A-00/15653
- WO-A-97/44134
- WO-A-98/12559
- WO-A-99/52625
- US-A- 5 449 754

## Beschreibung

Die Erfindung betrifft Verfahren und Vorrichtungen zum Aufbringen von Substanzen auf einen Träger, insbesondere von Monomeren für die kombinatorische Synthese von Molekülbibliotheken wie sie bei der Erfassung optischer Eigenschaften, insbesondere von Lumineszenz-Reaktionen und Brechungsverhalten, von auf dem Träger gebundenen Molekülen, verwendet werden.

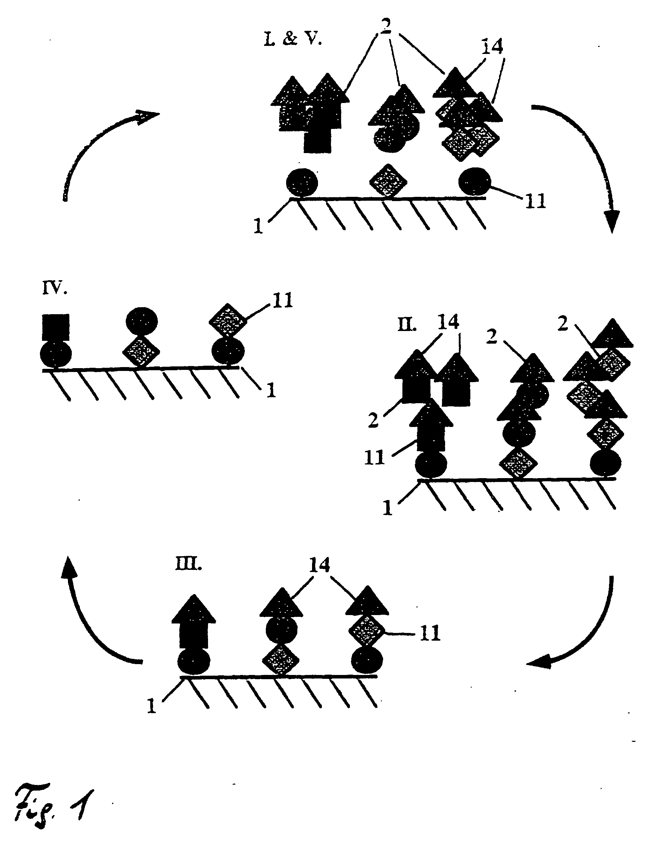

Dabei bezeichnet hier der Begriff "Molekülbibliothek" die Gesamtheit von vielen unterschiedlichen, an definierten Orten auf einem Träger gebundenen Molekülen, wobei die unterschiedlichen Moleküle möglichst kompakt angeordnet sind. Die Molekülbibliotheken, auf die sich die Erfindung bezieht, sind dabei insbesondere durch die kombinatorische Synthese einer begrenzten Zahl von Monomeren entstanden. In Fig. 1 wird das Prinzip der kombinatorischen Synthese schematisch erklärt.

Der Begriff "hochkomplex" bezeichnet Molekülbibliotheken mit mehr als 10³ unterschiedlichen Vertretern, insbesondere jedoch Molekülbibliotheken mit mehr als 10⁵ unterschiedlichen Vertretern.

Die genannten komplexen Molekülbibliotheken können besonders vorteilhaft auf einen zweidimensionalen Träger aufgebracht werden, wobei dann jedem unterschiedlichen Mitglied der Molekülbibliothek ein ortsgenau definierter Platz auf dem Träger zugewiesen werden kann. Dies bedeutet, dass eine ortsgenau definierte Reaktion, z.B. eine Färbereaktion, genaue und eindeutige Rückschlüsse auf den Träger-gebundenen Reaktionspartner zuläßt. Die ortsgenau definierten Plätze mit den definierten Mitgliedern der genannten Molekülbibliothek werden hier auch als Spots bezeichnet, die Gesamtheit der Molekülbibliothek auf dem zweidimensionalen Träger wird auch als Array bezeichnet.

Der zweidimensionale Array kann eine glatte Oberfläche besitzen und im wesentlichen undurchdringlich für die verwendeten Lösungsmittel sein. Er kann aber auch eine damit verglichen poröse Struktur aufweisen, sodass sich den verwendeten Lösungsmitteln und den zu koppelnden Substanzen eine dritte Dimension erschließt. Träger dieser Art sind insbesondere dann unabdingbar, wenn die Signalstärke verglichen mit einem Träger mit glatter Oberfläche erhöht werden soll. Dies ist insbesondere dann der Fall, wenn z. B. ein Array von Peptiden mit dem Blutserum eines Patienten gefärbt werden soll und dabei auch die Bindesignale von relativ schwach konzentrierten Antikörper-Reaktivitäten mit erfaßt werden sollen.

Im folgenden bezeichnen somit die synonymen Begriffe "zweidimensionaler Träger" oder "Array" sowohl Träger, bei denen unterschiedliche Moleküle im wesentlichen in nur 2 Dimensionen angeordnet sind, als auch poröse Träger, bei denen die unterschiedlichen Moleküle in einer zusätzlichen dritten Dimension vorliegen, also nicht mehr (im wesentlchen zweidimensionale) Arrays im eigentlichen Sinne darstellen.

Der Begriff "fester Aggregatszustand" beinhaltet auch unterkühlte Flüssigkeiten.

Der Begriff "Eigenschaften" wird im weitesten Sinne verstanden und soll nicht nur die für bestimmte Moleküle charakteristischen Eigenschaften, wie zum Beispiel deren Massenspektrogramm, umfassen, sondern zum Beispiel auch die Fähigkeit, überhaupt - nämlich durch das bloße Vorhandensein - eine bestimmte Reaktion zu zeigen, so dass die Erfindung also auch solche Verfahren und Vorrichtungen betrifft, bei denen aus einer bestimmten optischen Reaktion zunächst nur auf das bloße Vorhandensein eines Stoffes - nicht aber dessen Art - geschlossen werden soll (wobei dann die Art des Stoffes zum Beispiel aus dessen Position auf dem Träger ermittelt wird).

Unter dem Begriff "biologische" Moleküle werden hier alle Arten von in der Biologie, der Pharmazie und der Medizin besonders relevanten Molekülen verstanden, also z.B. Peptide, D-Peptide, L-Peptide und Mischungen daraus, natürlich vorkommende Oligonukleotide, ihre Spiegelbilder und Mischungen daraus, künstlich derivatisierte Oligonukleotide, wie sie zum Aufbau von Aptameren eingesetzt werden, Oligosaccharide und Modifikationen der genannten Moleküle. Insbesondere modular aufgebaute Oligomere, die nicht in der Natur vorkommen, können dabei besondere pharmakologische Relevanz besitzen. Besonders seien in diesem Zusammenhang auch mit Hilfe chemischer Kombinatorik herzustellende nichtnatürliche Substanzen erwähnt, die als Liganden von biologischen Molekülen eingesetzt werden können, insbesondere organische Verbindungen, Steroidderivate usw. Aus einer Vielzahl solcher Moleküle können spezifische Binder für ein natürlich vorkommendes Molekül isoliert werden, die die Aktivität dieses Moleküls modifizieren. Da diese Binder dann jedoch oft von natürlich vorkommenden Verdauungsenzymen nicht gespalten werden können, eignen sie sich in besonderem Maße für den Einsatz als Therapeutikum.

Zur Synthese hochkomplexer Molekülbibliotheken sind unterschiedliche Verfahren und Vorrichtungen bekannt, die jedoch bestimmte Nachteile aufweisen. So benötigen die bekannten Verfahren zu ihrer Durchführung aufwendige und teure Spezialgeräte und sind vergleichsweise langsam im Auslesen eines Lumineszenzsignals. Insbesondere wenn - was, wie nachfolgend noch erläutert wird, aus unterschiedlichen Gründen vorteilhaft ist - sehr viele unterschiedliche Molekülgruppen auf einem gemeinsamen Träger angeordnet und einzeln zu untersuchen sind, muß zum Anfahren der einzelnen Molekülgruppen eine sehr aufwendige Mechanik verwendet werden, die nicht nur teuer und störanfällig ist, sondern die auch bei höchster Präzisionsarbeit immer Fertigungstoleranzen aufweist, die um etliche Größenordnungen höher liegen, als die für eine Untersuchung ausreichende minimale Größe der Molekülgruppen. Dadurch ist die Anzahl der auf einem Träger maximal unterzubringenden Moleküle bzw. Molekülgruppen bei den bekannten Verfahren und Vorrichtungen begrenzt und liegt etwa in der Größenordnung einiger 10⁵ Molekülgruppen. Insbesondere für bestimmte Blutserums- oder DNA-Analysen wäre es jedoch wünschenswert, wenn auf einem Träger etwa 10⁸ bis 10⁹ Moleküle aufgebracht und untersucht werden könnten.

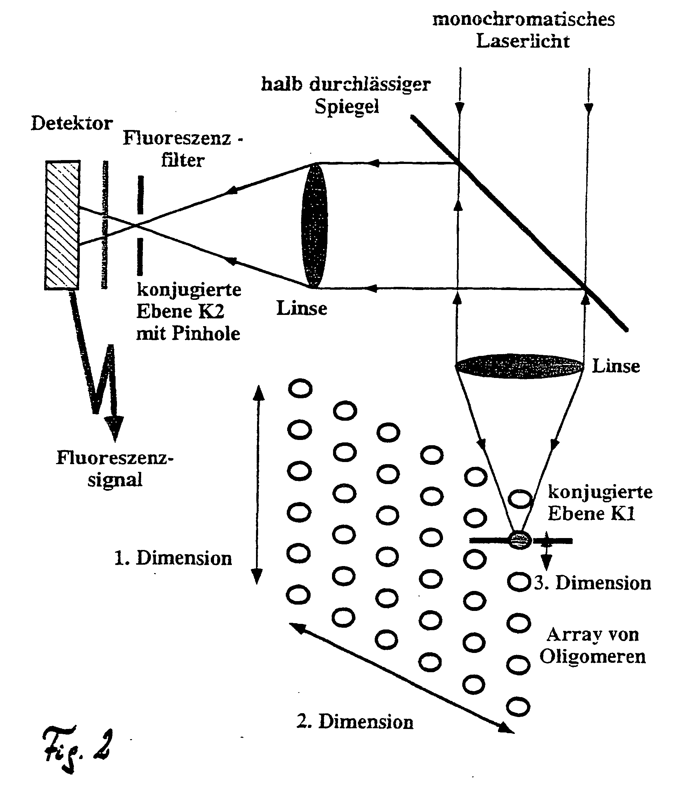

In Fig. 2 ist das Prinzip des konfokalen Lasermikroskops dargestellt, das für das Auslesen der gängigen, insbesondere der lithographischen Synthesemethoden verwendet wird. Dabei wird ersichtlich, dass bei diesem Auslesemechanismus jeder einzelne Punkt des Arrays in allen 3 Dimensionen durchsucht werden muß, was entweder Zeit oder Genauigkeit kostet.

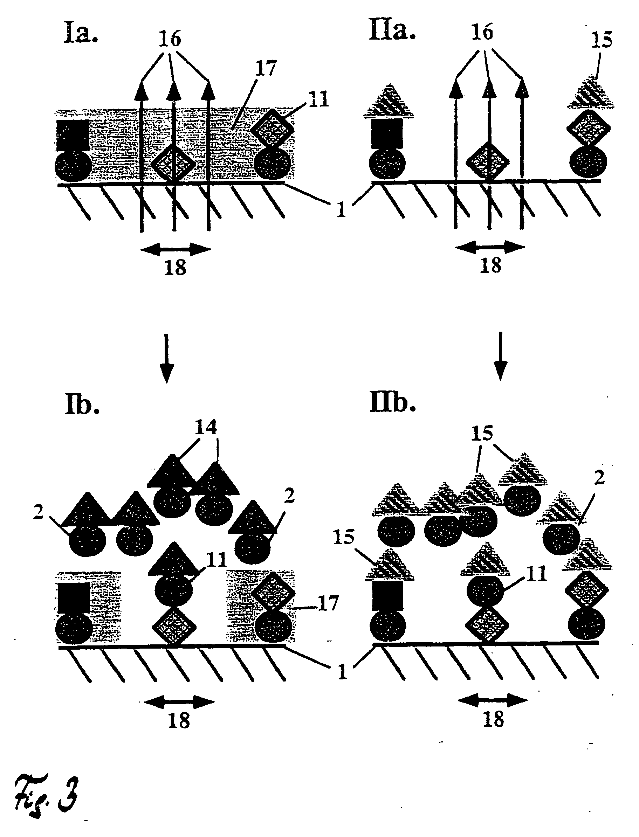

Zum Aufbringen der Moleküle auf die jeweiligen Träger, insbesondere auf sogenannte "Diagnostik-Chips", sind lithographische Methoden bekannt (Fig. 3), wobei jedoch - ähnlich wie bei der späteren Untersuchung - die schwierige exakte Zuordnung von Molekül und wiederholbar gezielt anfahrbarer Trägerposition die Zahl der maximal aufbringbaren Moleküle begrenzt, denn es genügt nicht, sehr viele unterschiedliche Moleküle dicht gepackt auf einem Träger anzuordnen, ohne jedoch wiederholbar genau zu wissen, welche Moleküle sich an welcher Position auf dem Träger befinden. Insbesondere ist es bei den bekannten Verfahren und Vorrichtungen ein Problem, sehr viele Lumineszenzreaktionen auf einem Träger in angemessener Zeit und gleichzeitig auch sehr genau auszulesen. Bei den mit den hier betroffenen Verfahren und Vorrichtungen vorteilhaft durchführbaren Färbeuntersuchungen, bei denen ein zu untersuchender Stoff auf den Träger, auf dem zuvor bereits unterschiedliche Moleküle verankert worden sind, aufgebracht wird, sollen Rückschlüsse auf die in dem zu untersuchenden Stoff vorhandenen Substanzen, wie z.B. bestimmte Antikörper in einem Blutserum, daraus gezogen werden, mit welchen der auf dem Träger verankerten Moleküle der Stoff bzw. dessen Bestandteile Bindungen eingegangen sind, so dass man sehr genau wissen muß, welches Molekül sich wo auf dem Träger befindet.

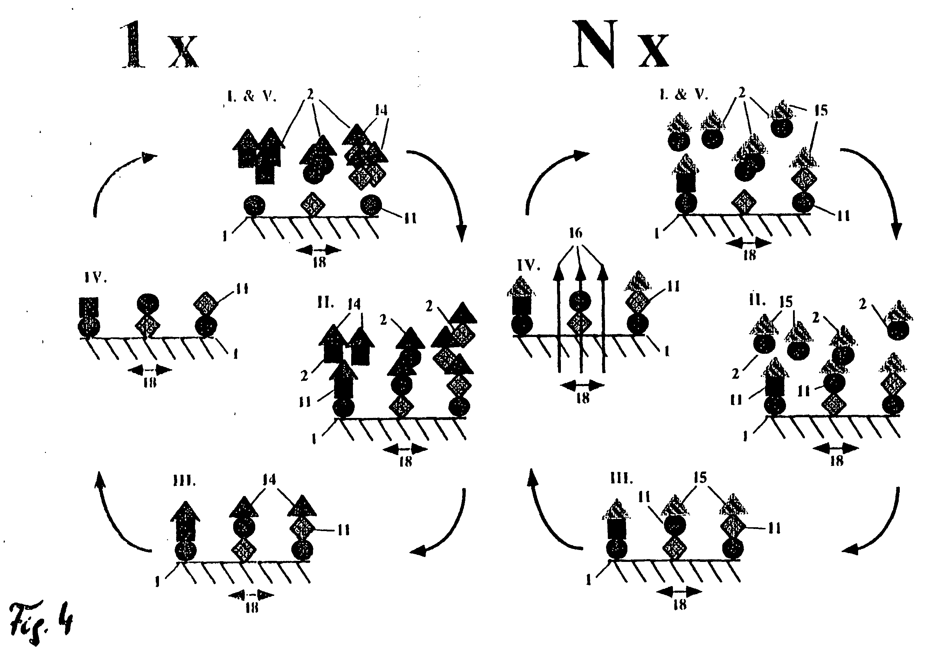

Darüberhinaus weisen alle bekannten lithographischen Methoden (und einige andere Methoden, wo beispielsweise die ortsgenaue Synthese durch die steuerbare Abstoßung oder Anziehung elektrisch geladener Monomere erreicht wird) einen weiteren, prinzipiellen Nachteil auf: Für jeden der unterschiedlichen Monomere muß gesondert fast der gesamte Kopplungszyklus durchlaufen werden, d.h. jede Art von Monomer wird aufgebracht, gekoppelt und überschüssige Monomere weggewaschen, gefolgt von der nächsten Art von Monomer, so dass beispielsweise bei der kombinatorischen Peptidsynthese Schicht für Schicht jeweils 20 Kopplungszyklen durchlaufen werden müssen. Dieser Nachteil ist schematisch in Fig. 4 dargestellt. Damit benötigen diese Methoden für die Synthese einer komplexen Pentapeptidbibliothek 100 Kopplungszyklen, wobei dem Fachmann sofort einsichtig ist, dass dies beim heutigen Stand der Technik aufgrund der bei jedem Kopplungszyklus zu erwartenden Artefakte zu schwerwiegenden Qualitätsproblemen der resultierenden Molekülbibliotheken führt, sodass solcherart hergestellte Pentapeptidbibliotheen faktisch unbrauchbar sind.

Dies ist auch der Grund, warum die lithographischen Verfahren bisher fast nur für die Synthese von Oligonukleotidarrays verwendet werden, da dabei nur 4 verschiedene Monomere an den Träger gekoppelt werden müssen.

Ein weiterer Nebeneffekt ist die vergleichsweise schlechte Chemikalienausbeute der lithographischen Methoden, da für jede einzelne Kopplungsreaktion der gesamte Träger gleichmäßig mit den reaktiven Monomeren bedeckt werden muß.

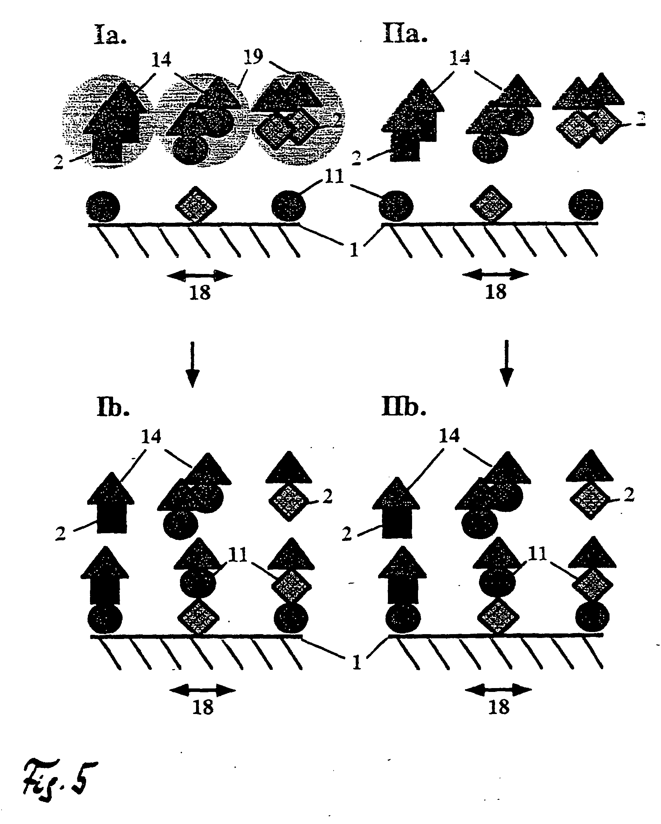

Neben den lithographischen Methoden sind auch eine Vielzahl von Druckmethoden bekannt, mit denen kombinatorische Synthesen durchgeführt werden können (Fig. 5, IIa & IIb).

WO-A-98/12559 beschreibt räumlich adressierbare kombinatorische Arrays im CD-ROM-Format, mit denen hohe Dichten von diskreten Spots verschiedener Substanzen erreicht werden.

US-A-5 449 754 offenbart die Verwendung der Tintenstrahltechnologie zur Erzeugung von Anordnungen kombinatorischer Substanzbibliotheken auf festen Trägern.

Beide Dokumente erwähnen nicht das technische Merkmal solider Transporteinheiten.

WO-A-97/44134 beschreibt eine Vorrichtung und ein Verfahren auf der Basis von Tintenstrahlern, das eine hohe Dichte von chemischen Individuen zur kombinatorischen Synthese auf entsprechenden Trägern erzeugt.

Die Verwendung von diskreten Transporteinheiten im Sinne der vorliegenden Erfindung und die Option Laser werden nicht erwähnt.

Bisher erreicht jedoch keines dieser Verfahren die hohe Auflösung der lithographischen Verfahren. Der Grund dafür liegt vor allem in der hohen Diffusionsrate der relativ kleinen Monomere in Lösung. Da sowohl für die Kopplungsreaktion der Monomere an den Träger, wie auch für das ortsgenaue Aufbringen der Monomere auf den Träger immer eine gewisse Zeit benötigt wird, begrenzt die hohe Diffusionsrate somit die erreichbare Kompaktheit der durch kombinatorische Synthese hergestellten Molekülbibliotheken und damit auch ihre Komplexität.

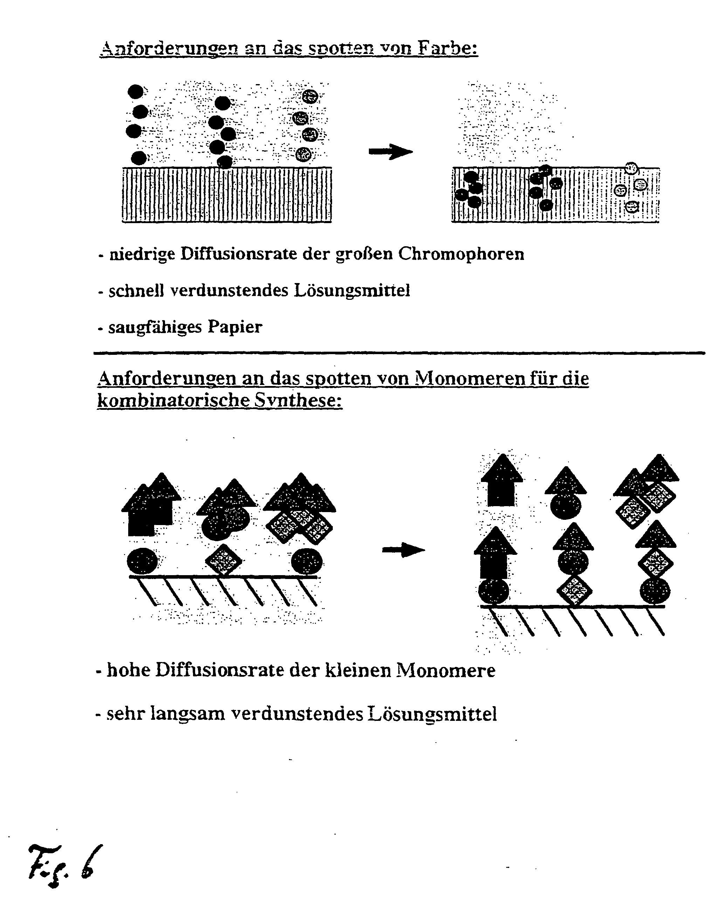

Ein Vergleich mit einem normalen Farbtintenstrahldrucker soll dieses Argument verdeutlichen (Fig. 6): Die Brillianz der Farbausdrucke von Farbtintenstrahldruckern wird dadurch erreicht, dass die Diffusion der verschiedenen Farbpartikel so gering wie möglich gehalten wird. Bewirkt wird dies durch die im Vergleich zu den oben genannten Monomeren enorme Größe der Farbpartikel und dadurch, dass die aufgedruckte Tonerflüssigkeit schnell flüchtige Substanzen enthält, sodass die Farbpartikel sehr schnell ausgefällt werden. Zusätzlich werden spezielle, stark saugfähige Hochglanzpapiere verwendet.

Diese meist kompliziert aufgebauten Papiere eignen sich in der Regel nicht als Träger für eine Molekülbibliothek und auch die beiden anderen Punkte widersprechen den Anforderungen an das Koppeln einer möglichst dicht gepackten Molekülbibliothek an den Träger:
1. Die Monomere für die kombinatorische Synthese sind sehr viel kleiner als die normalerweise verwendeten Farbchromophoren eines Farbtinten-Strahldruckers, alleine diese Tatsache erhöht die Diffusionsrate enorm.
2. Die aufgedruckten Monomere dürfen nicht nur nicht in leicht flüchtigen Lösungsmitteln gelöst sein. Es ist sogar kaum machbar ein Lösungsmittel zu finden, das in den angestrebten Mengen im Bereich von Nanolitern nicht zu schnell verdampft, denn damit würden sich die Konzentrationen der Kopplungspartner in unerwünschter Weise verändern, denn die Kopplungsreaktion an den Träger (und das ortsgenaue Aufbringen der Monomere auf den Träger) braucht eine gewisse Zeit.

Dies ist der Grund, warum alle bisher verwendeten Spotmethoden fehleranfällig und teuer sind, sobald sie in kleinere Dimensionen vordringen. In diesen Dimensionen besteht immer die Gefahr, dass die aufgebrachten Spots verlaufen, wegspritzen, die Monomere zu weit diffundieren oder das Lösungsmittel teilweise oder ganz verdunstet.

Davon ausgehend liegt der Erfindung die Aufgabe zugrunde, ein Verfahren und eine Vorrichtung anzugeben, mittels welcher die genannten Nachteile bei der Synthese von Molekülbibliotheken auf Trägern überwunden werden können.

Zur Lösung der Aufgabe wird ein Verfahren zur parallelen Synthese hochkomplexer Molekülbibliotheken vorgeschlagen, das dadurch gekennzeichnet ist, das die für die kombinatorische Synthese verwendeten Monomere vor oder nach dem Transfer auf den Träger in einem ersten Lösungsmittel gelöst werden, das unterhalb von -5°C, vorzugsweise unterhalb von +20°C in einem festen Aggregatszustand vorliegt und das einen Verdampfungspunkt > 100°C, vorzugsweise > 150°C aufweist. Weiteres Merkmal des Verfahrens ist, dass der genannte feste Aggregatszustand vorzugsweise kurzzeitig durch die Zuführung von Energie oder durch die Zuführung eines zweiten Lösungsmittels während der eigentlichen Kopplungsreaktion der Monomere an den Träger wiederum in einen flüssigen, vorzugsweise Gel-artigen Aggregatszustand umgeändert wird.

Dadurch wird
1. die Diffusion der Monomere deutlich eingeschränkt und
2. verhindert, dass das verwendete genannte erste Lösungsmittel während des Aufbringens der Monomere oder während der Kopplungsreaktion teilweise oder ganz verdunstet.

Dies ist insbesondere dann sehr wichtig, wenn das ortsgenaue Aufbringen der verschiedenen Monomere auf den Träger längere Zeit dauert, was beispielsweise dann der Fall ist, wenn hochkomplexe Peptidbibliotheken mit Hilfe eines Tintenstrahldruckers durch kombinatorische Synthese von unterschiedlichen Aminosäurederivaten hergestellt werden sollen.

Das Verfahren kann vorteilhaft so durchgeführt werden, dass in einem repetitiven Prozess die genannten Partikel oder Substanzen wiederholt ortsgnau auf den Träger aufgebracht werden, jeweils gefolgt von dem oben beschriebenen Beweglich-machen der immobilisierten Substanz, dem Koppeln der Substanz an den Träger, dem Wegwaschen nicht-gekoppelter Substanzen und dem Abspalten der temporären Schutzgruppe. Wird dabei ein abgewandelter Farblaserdrucker oder ein Farblaserkopierer verwendet, so kann vorteilhaft so vorgegangen werden, dass der Träger während des gesamten repetitiven Prozesses an der Trägerwalze oder der Transferwalze des Druckers bzw. Kopiers fixiert bleibt.

Statt eines einzelnen Lasers kann natürlich auch ein Array aus einer Anzahl von gezielt ansteuerbaren Lichtquellen, insbesondere ein Array von Mikrolasern verwendet werden. Durch die Einwirkung der elektromagnetischen Wellen auf die Partikel oder den Trägern werden diese ortsgenau elektrostatisch aufgeladen oder aufgeheizt, wodurch der ortsgenaue Transfer oder das ortsgenaue Fixieren der genannten Partikel bewirkt wird.

Enthalten die Partikel Vorstufen von für eine kombinatorische Synthese geeigneten Monomeren, Dimeren oder Trimeren, so können durch ein oder mehrere weitere Zyklen von Kopplungsreaktionen die an den Träger gebundenen Moleküle um weitere Monomere, Dimere oder Trimere verlängert werden. Auch ist es möglich, durch ein oder mehrere weitere Zyklen von nicht notwendigerweise identischen Reaktionen die an den Träger gebundenen Moleküle zu modifizieren. Nach erfolgter Synthese können die Schutzgruppen von den synthetisierten Oligomeren abgespalten werden, wobei die synthetisierten Moleküle an den Träger gebunden bleiben.

Die Partikel und/oder die immobilisierte Substanz kann geschmolzen oder durch eine zweite Substanz gelöst oder in einen Gel-artigen Zustand gebracht werden.

Vorteilhaft kann das Beweglich-machen der genannten immobilisierten Substanz durch das Einwirken von elektromagnetischen Wellen, insbesondere von Laserlicht, oder durch das Anlegen einer elektrischen Spannung, oder durch die Zufuhr thermischer Energie, oder durch die Zugabe eines Lösungsmittels erfolgen. Dabei kann sich das Beweglich-machen der immobilisierten Substanz auf ausgewählte Bereiche beschränken. Nicht beweglich gemachte oder nicht gekoppelte Substanzen können mit einem Lösungsmittel, vorzugsweise einem erwärmten Lösungsmittel, vom Träger gewaschen oder mechanisch, insbesondere mit Hilfe eines Luftstroms vom Träger entfernt werden.

Als Träger können unterschiedliche Materialien verwendet werden. Insbesondere können Polystyrolfolien, Papier, CDs, MODs, DVDs oder FMDs verwendet werden.

Die mit dem erfindungsgemäßen Verfahren hergestellten Träger können auf unterschiedlichste Weise bei wissenschaftlichen, insbesondere medizinischen Untersuchungen verwendet werden. Dazu wird der Träger üblicherweise mit einer zu untersuchenden Flüssigkeit in Kontakt gebracht. Bei dieser Flüssigkeit kann es sich zum Beispiel um Blut, Blutserum, Urin, Faeces, Lymphe, Speichel, Fruchtblasenflüssigkeit, Magensaft, Erbrochenes, Schweiß, Samenflüssigkeit, Muttermilch, Tränenflüssigkeit, um eine Antikörper enthaltende Flüssigkeit oder um einen Extrakt aus den genannten Flüssigkeiten handeln. Handelt es sich bei der zu untersuchenden Flüssigkeit um ein Blutserum, so kann dieses vorteilhaft mit einem für Immunglobuline spezifischen Nachweisreagenz, insbesondere für einen mit Immunglobuline des Typs IgE, IgM, IgG oder IaA in Kontakt gebracht werden. Es ist auch möglich, den Träger mit zu untersuchender DNA in Kontakt zu bringen. Soll DNA oder eine Immunglobuline enthaltende Flüssigkeit untersucht werden, so wird vorteilhaft so vorgegangen, dass die zu untersuchende Flüssigkeit oder die zu untersuchende DNA nach oder vor dem In-Kontakt-Bringen mit dem Träger mit einem mit Immunglobulinen oder mit DNA reagierenden, insbesondere Bindungen eingehenden Stoff in Kontakt gebracht wird. Dieser mit Immunglobulinen oder DNA reagierende Stoff kann vor dem In-Kontakt-Bringen mit der zu untersuchenden Flüssigkeit oder der zu untersuchenden DNA mit einem zur Lumineszenz anregbaren Stoff eingefärbt und/oder mit einem weiteren Stoff gekoppelt werden, der eine Lumineszenz hervorrufen kann, insbesondere also mit einem Enzym oder einer Vorstufe eines lumineszenten Stoffes. Bei dem genannten weiteren Stoff kann es sich vorteilhaft um ein Enzym, insbesondere Meerettich-Peroxidase, Alkalische Phosphatase, Beta-Galaktosidase, Glukose-Oxidase, Lactat-Dehydrogenase oder Luziferase, oder eine Bindung von Enzymen handeln.

Als zur Lumineszenz anregbarer Stoff wird zweckmäßigerweise ein durch Einstrahlung von elektromagnetischen Wellen, insbesondere von Laserlicht oder Licht aus Leuchtdioden zur Fluoreszenz anregbarer Farbstoff verwendet.

Die auf diese Weise gewonnen Untersuchungsergebnisse können vorteilhaft zur systematischen Klassifizierung und Segmentierung von pathologischen Mustern, insbesondere zur Bestimmung von diagnostischen Markern, verwendet werden, in dem die genannten Untersuchungsverfahren bei einer Vielzahl von Testpersonen durchgeführt werden und in den Testergebnissen vorhandene Abweichungen von der Normalverteilung ermittelt werden.

Beispielsweise ist es möglich, von jeder Testpersonen jeweils eine Probe Blutserum zu entnehmen und nach einem der genannten Verfahren zu untersuchen. Die Abweichung von der Normalverteilung kann beispielsweise für Testpersonen ermittelt werden, die vor oder nach der Gewinnung des Blutserums an Krebs, insbesondere an einzelnen Krebsarten, an der Parkinsonschen Krankheit, an Multipler Sklerose, an der Alzheimerschen Krankheit, an einer Infektionskrankheit, an einer Autoimmunkrankheit, insbesondere an Morbus Crohn, erkrankt sind oder einen Herzinfarkt oder Schlaganfall erlitten haben oder Allergiker waren oder wurden.

Die auf diese Weise gewonnenen Testergebnisses können automatisch klassifiziert werden, um so diagnostische Muster zu bestimmen, in dem die Signale mit Strukturparametern der entsprechenden Moleküle der Molekülbibliothek in Beziehung gebracht werden, sodass Korrelationen aufgefunden werden, insbesondere solche Korrelationen, die eine diagnostische Beurteilung von Mustern unbekannter Proben ermöglichen. Es kann auch nach Korrelationen gesucht werden, die es erlauben, Strukturmerkmale der aufgefundenen Moleküle zu ermitteln. Die dabei definierten Strukturmerkmale der aufgefundenen Moleküle können als Leitstrukturen für die Entwicklung funktionshomologer anderer Moleküle, insbesondere therapeutisch anwendbarer Moleküle, dienen. Dabei kann vorteilhaft ein Peptidarray verwendet werden, das bekannte menschliche Genprodukte durch einander überlappende Peptide abdeckt.

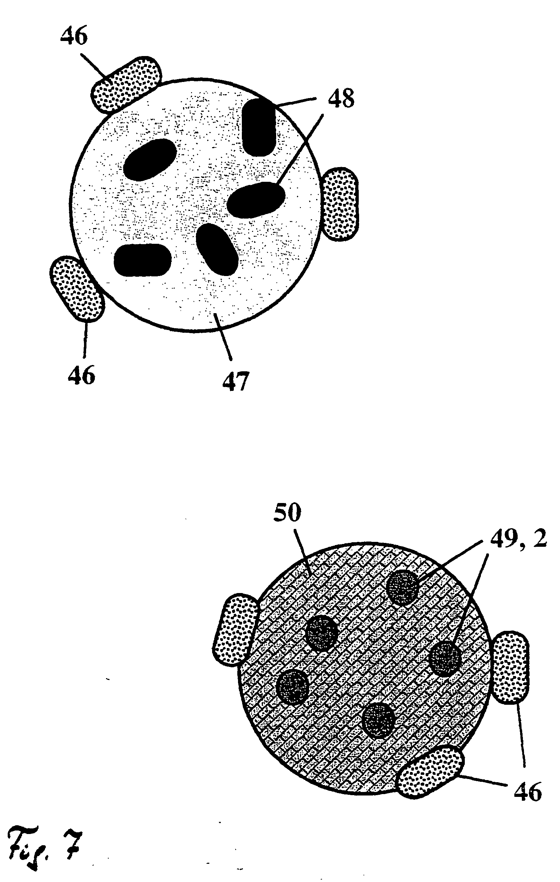

In einem zusätzlichen Verfahren werden die genannten Monomere für die kombinatorische Synthese in 0,2µm bis 200µm, vorzugsweise 2µm bis 40µm große Monomer-Tonerpartikel eingeschlossen, die bei Raumtemperatur den Aggregatszustand fest annehmen. Der Begriff Raumtemperatur beschreibt dabei eine Temperaturspanne zwischen -10°C und 80°C, vorzugsweise jedoch 0°C bis 40°C. Weiteres Kennzeichen dieser Partikel ist, dass sie mit dem genannten ersten Lösungsmittel einen im Bezug auf die Kopplungsreaktion inerten Bestandteil enthalten, dessen Aggregatszustand wie oben beschrieben geändert werden kann. Vorzugsweise enthalten die genannten Partikel darüber hinaus noch magnetische Bestandteile oder binden an Partikel, die magnetische Bestandteile enthalten. In Fig. 7 wird ein solcher Monomer-Tonerpartikel schematisch mit einem normalen Chromophoren-Tonerpartikel verglichen.

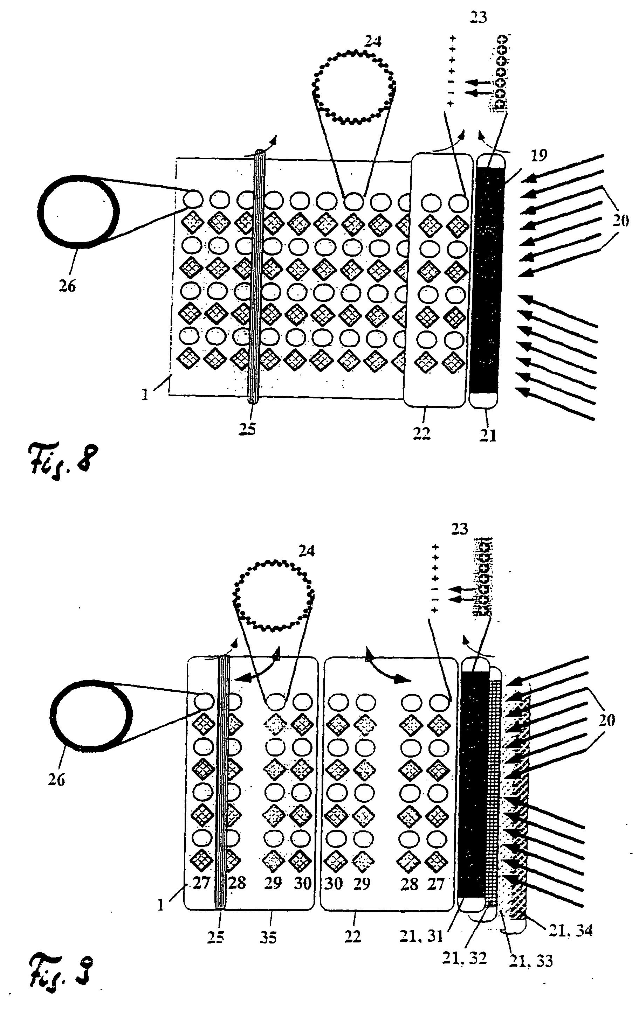

Der ortsgenaue Transfer der Partikel auf den Träger erfolgt anschließend mit einem weitgehend handelsüblichen Laserdrucker (Fig. 8) oder Laserkopierer, insbesondere einem Farblaserdrucker (Fig. 9) oder Farblaserkopierer, wobei der für den Transfer der Partikel verantwortliche Laser, insbesondere beim Laserkopierer auch durch einen 1-oder 2-dimensionalen Array von Mikrolasern ersetzt sein kann.

Die ortsgenau aufgebrachten Monomere werden anschließend wie oben beschrieben vom festen Aggregatszustand in einen flüssigen, vorzugsweise Gel-artigen Aggregatszustand überführt, wodurch eine ortsgenau definierte Kopplungsreaktion in Gang gesetzt wird.

Dadurch wird verglichen mit dem bisherigen Stand der Technik sehr einfach eine wesentlich kompaktere Anordnung der mit Hilfe kombinatorischer Synthesen erzeugten Moleküle auf einem Träger erreicht und damit die Herstellung von hochkomplexen, vergleichsweise Artefakt-freien Molekülbibliotheken.

Die technischen Kennzahlen eines Laserdruckers sollen dieses Argument noch weiter verdeutlichen:

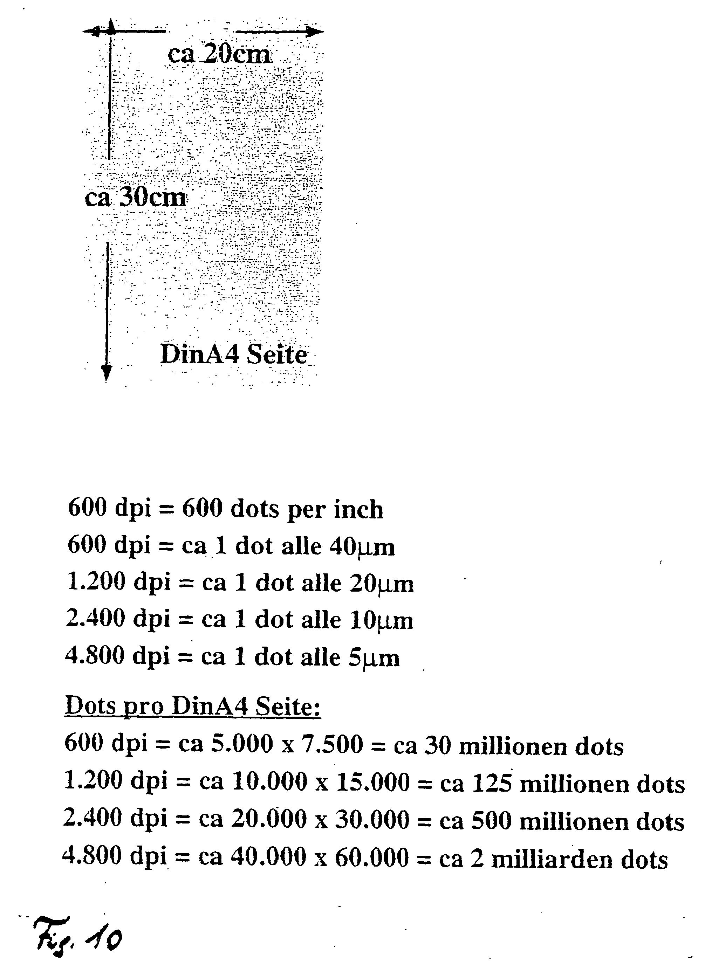

Handelsübliche Laserdrucker haben eine Auflösung der gedruckten Punkte von 600dpi (dots per inch). Das entspricht einem Durchmesser der aufgedruckten einzelnen Pixel von ca 40µm, bzw. ein solcher Laserdrucker setzt bei jedem normalen Druckvorgang pro DinA4 Seite (ca 20 x 30 cm) etwa 4500 x 7000 Punkte. Dies wiederum entspricht etwa 30 millionen Punkte pro DinA4 Seite (Fig. 10).

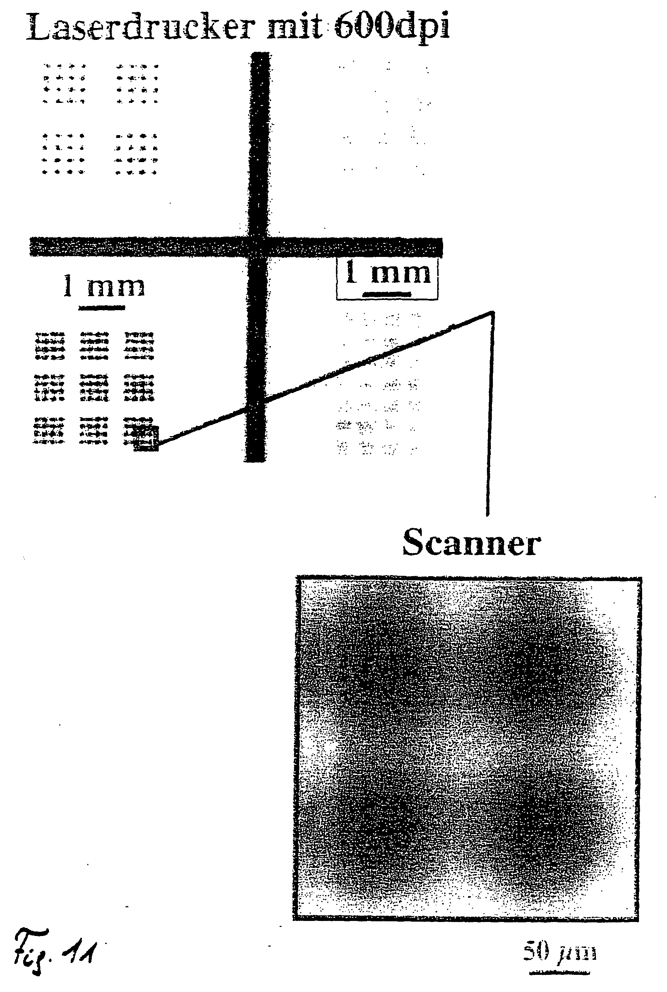

Fig. 11 zeigt, daß diese hohe Auflösung bei einem normalen Druckvorgang mit normalem Toner nahezu fehlerfrei und reproduzierbar "geerntet" werden kann. Tatsächlich zeigt auch die stärkste Vergrößerung von Fig. 11 keinen einzigen falsch gesetzten Pixel.

Damit können Millionen von Spots auf einer DinA4-Seite separat identifizierbar untergebracht werden. Dies ist aber sicherlich noch lange nicht der Endpunkt einer offensichtlich rasanten technologischen Entwicklung. Die bereits jetzt schon käuflich erwerbbaren Laserdrucker mit einer Auflösung von 2.400dpi setzen etwa 500 Millionen Bildpunkte pro DinA4 Seite, womit noch sehr viel mehr Spots auf einer DinA4-Seite separat voneinander untergebracht werden können.

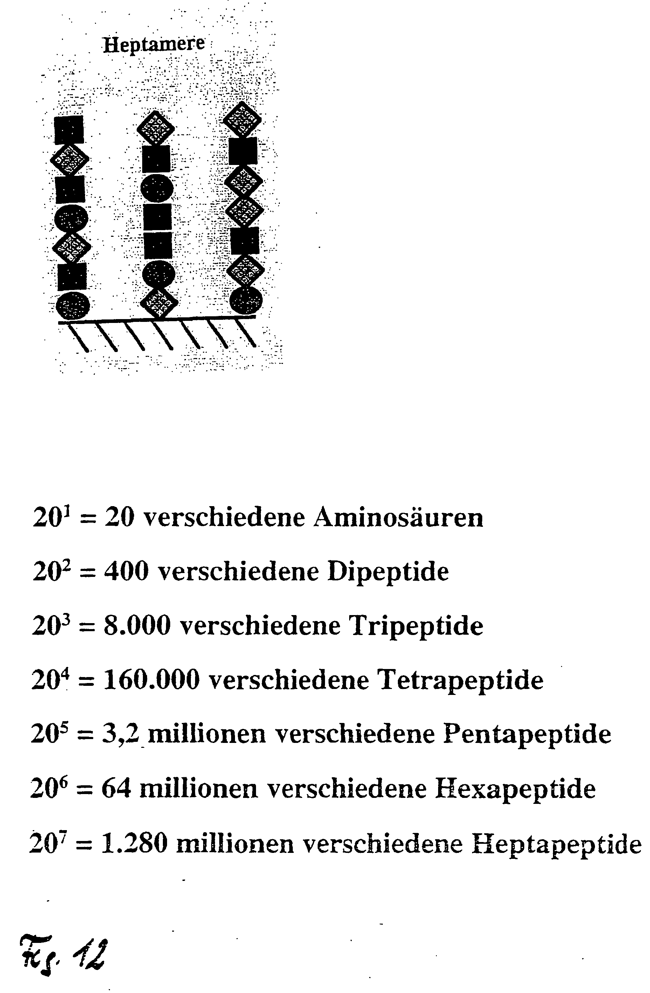

Damit dringen die handelsüblich erwerbbaren Laserdrucker in Zahlendimensionen vor, die die kombinatorische Synthese von Hexapeptid oder Pentapeptid-Bibliotheken mit allen möglichen Vertretern ermöglichen. In Fig. 12 sind die entsprechenden Zahlengrößen dargestellt.

In einem alternativen zusätzlichen Verfahren werden die genannten Monomere für die kombinatorische Synthese nach dem bekannten Stand der Technik in flüssigem Aggregatszustand ortsgenau auf den Träger aufgebracht, beispielsweise mit Hilfe eines im wesentlichen handelsüblichen Tintenstrahldruckers oder sonstiger Druckverfahren.

Dazu wird ein Lösungsmittelgemisch hergestellt, dem neben den genannten Monomeren für die kombinatorische Synthese und dem genannten ersten Lösungsmittel (beispielsweise Diphenylformamid) mindestens ein zweites Lösungsmittel (beispielsweiseN-Methyl-Pyrrilidon, Dichlormethan, Dimethylformamid, Methanoloder Isopropanol) beigemischt wird, das die genannten Komponenten bei Raumtemperatur vom Aggregatszustand fest in den Aggregatszustand flüssig überführt (d.h. löst). Der Begriff Raumtemperatur beschreibt dabei eine Temperaturspanne zwischen -10°C und 80°C, vorzugsweise jedoch 0°C bis 40°C.

Weiteres Kennzeichen des genannten Lösungsmittelgemisches ist, dass bei Raumtemperatur das genannte zweite Lösungsmittel verglichen mit dem genannten ersten Lösungsmittel leicht flüchtig ist, d.h., dass sich der Verdampfungspunkt beider genannter Lösungsmittel um > 80°C voneinander unterscheidet.

Dabei bewirkt das Verdunsten eines Teils des genannten zweiten Lösungsmittels, dass das genannte erste Lösungsmittel zusammen mit den darin gelösten Monomeren für die kombinatorische Synthese zunächst aufkonzentriert wird und anschließend vom Aggregatszustand flüssig wieder in den Aggregatszustand fest oder Gel-artig überführt wird, ohne dass die Monomere sich durch Diffusion weit von dem ursprünglichen Auftragsort entfernen können. Der genannte Träger wird dabei vorzugsweise bei einer > 10°C tieferen Temperatur gehalten als der Vorratsbehälter mit dem genannten Lösungsmittelgemisch. Der schwer flüchtige Lösungsmittelanteil kann dabei auch vor dem ortsgenauen Aufbringen des genannten Gemischs ganz oder teilweise auf den Träger aufgezogen werden. Die verwendeten Lösungsmittel müssen dabei in Bezug auf die Kopplungsreaktion inert sein.

Die ortsgenau aufgebrachten Monomere werden anschließend wie oben beschrieben vom festen Aggregatszustand in einen flüssigen, vorzugsweise Gel-artigen Aggregatszustand überführt, wodurch eine ortsgenau definierte Kopplungsreaktion in Gang gesetzt wird.

Auch dadurch wird verglichen mit dem bisherigen Stand der Technik sehr einfach eine wesentlich kompaktere Anordnung der mit Hilfe kombinatorischer Synthesen erzeugten Moleküle auf einem Träger erreicht und damit die Herstellung von hochkomplexen, vergleichsweise Artefakt-freien Molekülbibliotheken.

Die Erfindung umfaßt Vorrichtungen, mit denen Monomere für die kombinatorische Synthese von Träger-gebundenen Molekülbibliotheken, insbesondere von Peptid oder Oligonukleotidbibliotheken ortsgenau aufgebracht werden können. Die Kopplung der einzelnen Schichten von aufgebrachten Monomeren erfolgt dabei wie weiter oben beschrieben.

Die genannten Monomere werden dabei wie weiter unten beschrieben in Partikel eingebunden.

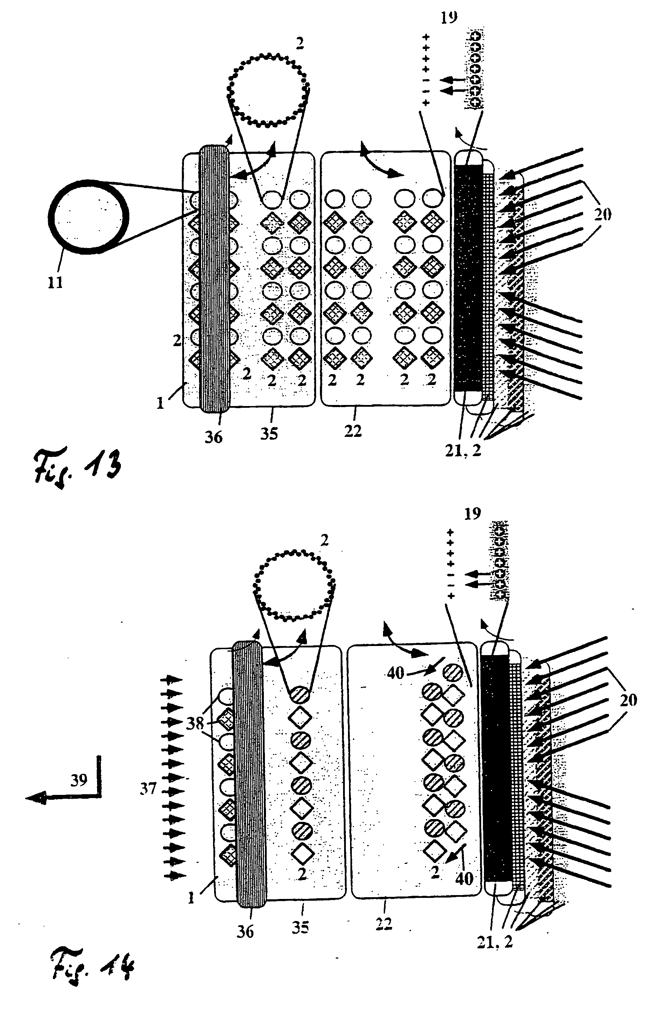

Der ortsgenaue Transfer der genannten Partikel auf den Träger erfolgt anschließend mit einer Vorrichtung (Fig. 13 & 14), die auf einem im wesentlichen handelsüblichen Laserdrucker oder Laserkopierer, insbesondere einem Farblaserdrucker oder Farblaserkopierer basiert. Dabei kann der für den Transfer der Partikel verantwortliche Laser, insbesondere beim Laserkopierer auch durch einen 1- oder 2-dimensionalen Array von Mikrolasern ersetzt werden.

Folgende Änderungen unterscheiden den genannten handelsüblichen Laserdrucker oder Laserkopierer von der genannten Vorrichtung:
1. Anstelle der üblichen Tonerpartikel werden die weiter unten beschriebenen, die genannten Monomere enthaltenden Partikel verwendet.
2. Es wird nicht nur eine Schicht von Monomer-Partikeln aufgebracht, sondern es werden mehrere Schichten von den die genannten Monomere enthaltenden Partikeln übereinandergedruckt.
3. Jeweils zwischen dem Aufbringen der genannten mehreren Schichten werden die genannten Monomere an den Träger gekoppelt, ungekoppelte Monomere vom Träger entfernt und die temporären Schutzgruppen von den Träger-gekoppelten Monomeren abgespalten.
4. Bis zum Aufbringen der letzten Schicht der genannten Monomere bleibt der Träger in einer exakten räumlichen Beziehung relativ zu dem für den Transfer der Partikel verantwortlichen Laser, wobei es genügt, dass diese räumliche Beziehung wiederholbar herstellbar ist. Damit können die genannten Schichten und innerhalb der Schichten die genannten unterschiedlichen Monomer-Partikeln, ortsgenau übereinander bzw. nebeneinander plaziert werden. Die genannte räumliche Beziehung kann dabei über einen Rückkopplungsmechanismus (Fig. 14) und oder durch eine exakte mechanische Kopplung (Fig. 13) der Trägerwalze oder der Transfereinheit mit der von dem Laser ionisierbaren Walze hergestellt werden. Beide Methoden können natürlich auch kombiniert werden.

Beispielsweise kann auf dem Träger oder auf der Trägerwalze (35) ein Raster von Positionsmarkierungen (Fig. 14, 38) aufgebracht werden, das von einer Scannerzeile (Fig. 14, 37) gelesen wird und mit einem eingespeicherten Raster verglichen (Fig. 14, 39) wird. Eine elektronische Verschiebung der Bildpunkte im Druckerspeicher genau um den Betrag der gemessenen Abweichung (Fig. 14, 40) ist dann Teil des genannten Rückkopplungsmechanismus.

Die Erfindung umfaßt erfindungsmäßig produzierte Materialien. Dazu zählen Monomer-Tonerpartikel, die von der genannten Vorrichtung, die auf einem im wesentlichen handelsüblichen Laserdrucker oder Laserkopierer basiert, ortsgenau auf den Träger aufgebracht werden können. Diese Monomer-Tonerpartikel unterscheiden sich wie folgt von den handelsüblichen Tonerpartikeln:
1. Anstelle der Chromophoren enthalten die Monomer-Tonerpartikel für die kombinatorische Synthese geeignete Monomere oder deren Derivate, insbesondere auch voraktivierte Monomere
2. Anstelle oder zusätzlich zu einem aufschmelzbaren Kunststoffanteil (z.B. Polystyrol) enthalten die Monomer-Tonerpartikel ein im Bezug auf die Kopplung der Monomere an den Träger inertes Lösungsmittel (z.B. Diphenylformamid), das bei Raumtemperatur den Aggregatszustand fest annimmt. Der Begriff Raumtemperatur beschreibt dabei eine Temperaturspanne zwischen -10°C und 80°C, vorzugsweise jedoch 0°C bis 40°C.

Weitere Kennzeichen der genannten Monomer-Tonerpartikel sind:
3. Ihre Größe beträgt 0,2µm bis 200µm, vorzugsweise jedoch 2µm bis 40µm im Durchmesser.
4. Die genannten Monomer-Tonerpartikel nehmen bei Raumtemperatur den Aggregatszustand fest an. Der Begriff Raumtemperatur beschreibt dabei eine Temperaturspanne zwischen -10°C und 80°C, vorzugsweise jedoch 0°C bis 40°C.
5. Weiteres Kennzeichen der genannten Monomer-Tonerpartikel ist, dass sie magnetische Bestandteile enthalten oder an Partikel binden, die magnetische Bestandteile enthalten.

Weitere erfindungsmäßig produzierte Materialien beinhalten Monomer-Tonerflüssigkeiten, die mit einem im wesentlichen handelsüblichen Tintenstrahldrucker oder Farbtintenstrahldrucker ortsgenau auf den Träger aufgebracht werden können.

Diese Monomer-Tonerflüssigkeiten unterscheiden sich wie folgt von den handelsüblichen Tonerflüssigkeiten:
1. Anstelle der Chromophoren enthalten die Monomer-Tonerflüssigkeiten für die kombinatorische Synthese geeignete Monomere oder deren Derivate, insbesondere auch voraktivierte Monomere.
2. Zusätzlich zu einem bei Raumtemperatur flüssigen ersten Lösungsmittelanteil (z.B. Isopropanol, Dimethylformamid, N-Methyl-Pyrrilidon, Dichlormethan) enthalten die Monomer-Tonerflüssigkeiten mindestens ein zweites im Bezug auf die Kopplung der Monomere an den Träger inertes Lösungsmittel (z.B. Diphenylformamid), das bei Raumtemperatur den Aggregatszustand fest annimmt. Der Begriff Raumtemperatur beschreibt dabei eine Temperaturspanne zwischen -10°C und 80°C, vorzugsweise jedoch 0°C bis 40°C. Das genannte zweite Lösungsmittel wird dabei bei Raumtemperatur von dem genannten ersten Lösungsmittel gelöst.

Kennzeichen des ersten Lösungsmittels ist, dass ein Schmelzpunkt und sein Verdampfungspunkt um mehr als 40 °C, vorzugsweise um mehr als 70 °C tiefer liegt, verglichen mit dem Schmelzpunkt und dem Verdampfungsunkt des zweiten Lösungsmittels.

Kennzeichen des ersten Lösungsmittels ist, dass es unterhalb von -80°C, vorzugsweise unterhalb von -20°C in einem festen Aggregatszustand vorliegt und dass es einen Verdampfungspunkt > 40°C, vorzugsweise > 70°C aufweist.

Kennzeichen des zweiten Lösungsmittels ist, dass es unterhalb von -5°C, vorzugsweise unterhalb von +20°C in einem festen Aggregatszustand vorliegt und dass es einen Verdampfungspunkt > 200°C, vorzugsweise > 250°C aufweist.

Kennzeichen des Gemischs aus den genannten mindestens 2 verschiedenen Lösungsmitteln zusammen mit den durch sie gelösten Monomeren ist, dass das Gemisch unterhalb von -20°C, vorzugsweise unterhalb von 0°C in einen festem oder Gel-artigen Aggregatszustand vorliegt.

Weitere erfindungsmäßig produzierte Materialien beinhalten die mit den genannten Verfahren, Materialien oder Vorrichtungen produzierten Molekülbibliotheken, insbesondere Peptid oder Oligonukleotidbibliotheken. Kennzeichen dieser Molekülbibliotheken ist:
1. dass sie durch die kombinatorische Synthese einer begrenzten Zahl von Monomeren entstanden sind,
2. dass sie als 2-dimensionales Array auf einem geeigneten derivatisierten Träger vorliegen, wobei die einzelnen Bestandteile der Molekülbibliothek ortsgenau zugeordnet werden können. Die Derivatisierung des Trägers erfolgt dabei mit dem Fachmann bekanntem Stand der Technik.

Als Träger für Moleküle, insbesondere biologische Moleküle, insbesondere zur Verwendung in einem der genannten Verfahren können erfindungsgemäß Träger Verwendung finden, die ein engmaschiges Netz von integrierten Positionsmarkierungen aufweisen, so dass es möglich wird, die Position einer z.B. mittels einer üblichen Mechanik mit einem Detektor auf dem Träger angefahrenen zu untersuchenden Stelle zu kontrollieren. Bevorzugt sind dabei die Positionsmarkierungen so ausgebildet, dass sie von einem optoelektronischen Abtastsystem erfaßbar sind.

Die Erfindung schafft damit völlig neue diagnostische Möglichkeiten und steigert insbesondere auch die Chancen, diagnostische Marker und Therapeutika zu finden. Werden z.B. vollständige 6mer Peptidbibliotheken mit einer größeren Anzahl von Patientenseren in Verbindung gebracht und z.B. mittels einer Färbereaktion daraufhin untersucht, an welchen Peptiden sich Bestandteile der Seren angelagert haben, so werden sich dabei Korrelationen zwischen Krankheit und gefärbten Peptiden ergeben. Dies liegt daran, dass jeder Mensch in seinem Blutserum ein sehr komplexes individuelles Muster von Antikörperreaktivitäten mit sich trägt, das insbesondere die Auseinandersetzung seines Immunsystems mit akuten, chronischen, verdeckten oder bereits überstandenen Krankheiten wiederspiegelt. Ein großer Teil der Antikörperreaktivitäten kann durch die spezifische Bindung an Penta- oder Hexapeptide definiert werden, wodurch bei der Analyse der Bindereaktivitäten an eine vollständige Penta- oder Hexapeptidbibliothek das o.g. individuelle Muster von Antikörperreaktivitäten in bisher nicht gekannter Komplexität bestimmt werden kann.

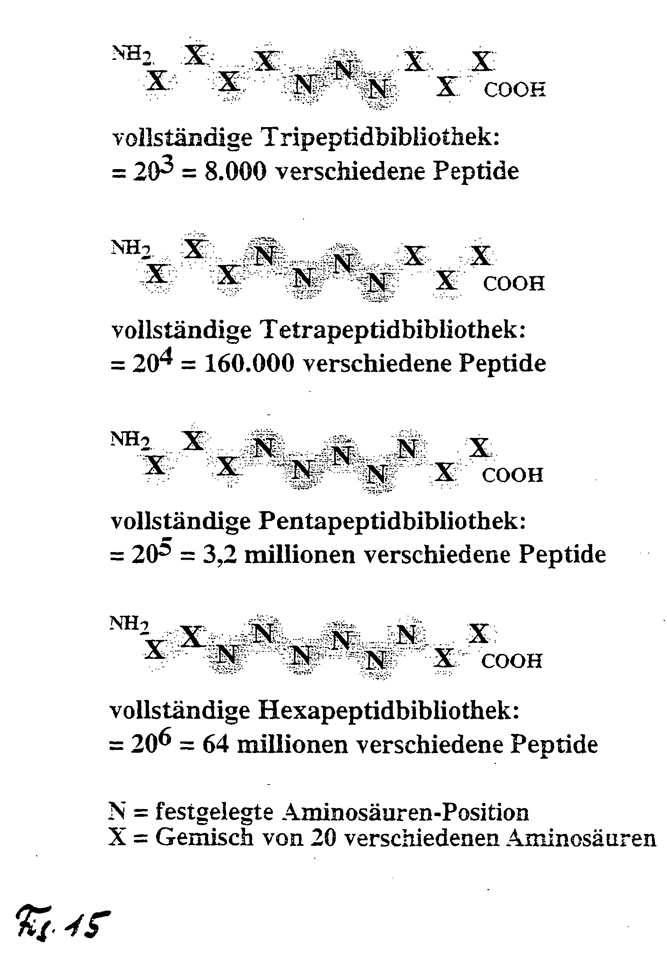

In Fig. 15 sind sogenannte vollständige Peptidbibliotheken erläutert. Jeder ortsgenau definierte Spot eines Arrays einer solchen vollständigen Peptidbibliothek repräsentiert dabei ein Peptidgemisch, das nur an den mit N bezeichneten Stellen pro Spot eine jeweils andere, definierte Sequenz trägt. Der Grund für das arbeiten mit diesen Peptidgemischen liegt darin, dass bei einer Antikörper-Peptidantigenreaktion das erkannte Peptidantigen eine gewisse Größe benötigt, damit es von dem Antikörper spezifisch erkannt werden kann. Da es jedoch mit dem heutigen Stand der Technik nicht möglich ist eine vollständige Decapeptidbibliothek mit 20¹⁰ verschiedenen Mitgliedern herzustellen, werden die genannten Gemische verwendet.

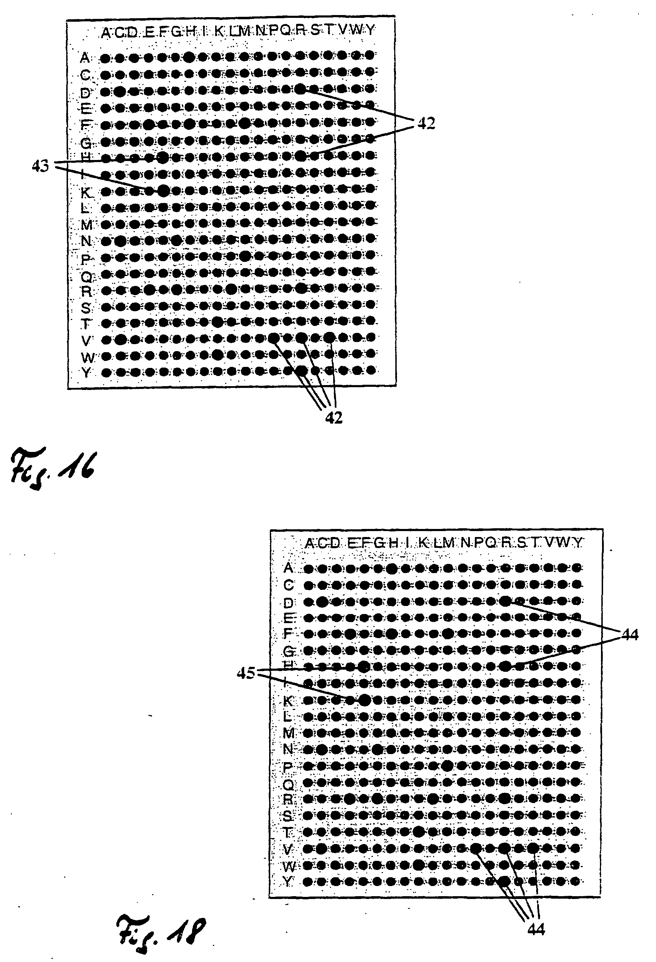

In Fig. 16 ist die vorteilhafte Verwendung solcher Arrays schematisch dargestellt (siehe auch das Beispiel (1.)). Die differentielle Färbung eines hochkomplexen Arrays von Peptiden mit Kontrollserum und dem Serum von Patienten ergibt dabei einerseits Reaktionspartner (und damit Peptidsequenzen), die von beiden Seren erkannt werden (Fig. 16, 42) und andererseits Peptide, die spezifisch von Patientenseren erkannt werden (Fig. 16, 43). Dies ermöglicht die Identifizierung von Patienten-spezifischen Färbemustern. Im angegebenen Beispiel (Fig. 16 und Beispiel (1.)) werden Peptide identifiziert (Fig. 16, 43), die exprimierten Genprodukten von Helicobacter pylori entsprechen, dem Verursacher von Magengeschwüren. Dies bedeutet, dass mit Hilfe solcher Arrays Krankheit und Peptidmuster miteinander korreliert werden können.

Neben der Epitopbestimmung von monoklonalen Antikörpern eignen sich die genannten vollständigen Peptidbibliotheken für das aufspüren von diagnostischen Markern durch die Korrelation des Serumprofils mit der diagnostizierten Krankheit, beispielsweise von Autoimmunkrankheiten oder Allergien (z.B. Rheuma, Heuschnupfen, Asthma, Lebensmittelallergien, Lupus erythematodes, juvenile Diabetis etc.), von Infektionskrankheiten (z.B. Grippe, grippale Infekte, AIDS, Hepatitis, Masern, Mumps, Hirnhautentzündung, Magengeschwüre, Malaria, Chagas-Krankheit etc.), von Krebserkrankungen (z.B. Lungenkrebs, Leberkrebs, Darmkrebs, Nierenkarzinom, Brustkrebs, Prostatakrebs, Glioblastom, Lymphome etc.) und insbesondere von Krankheiten bisher unbekannter oder zweifelhafter Ursache (z.B. Herzinfarkt, Schlaganfall, Parkinsonschen Krankheit, Multiple Sklerose, Alzheimersche Krankheit, Morbus Crohn, Creutzfeld-Jacobs Krankheit etc.).

Dabei ist dieses Verfahren nicht auf eine Krankheit beschränkt, es können damit auch sehr viele Krankheiten parallel diagnostiziert werden, oder umgekehrt mit Hilfe der aufgefundenen Peptide bisher unbekannten Krankheitsursachen nachgespürt werden.

Nicht zuletzt eigenen sich die genannten Arrays aber auch für die Suche nach Interaktionspartnern, beispielsweise von menschlichen oder viralen Genprodukten, mit denen man in diesem Fall die genannten vollständigen Bibliotheken anfärbt. Bei der Anfärbung mit gereinigten Viruspartikeln könnte man z.B. sehr schnell eines oder mehrere Bindemotive identifizieren und durch einen Vergleich der entsprechenden Peptidsequenzen mit den in den Datenbanken abgelegten humanen Peptidsequenzen die Eintrittspforte des Virus in menschliche Zellen identifizieren.

Längere Bindemotive, insbesondere die ebenfalls häufig vorkommenden mit einer helikalen Struktur, können durch Bibliotheken ermittelt werden, in denen nicht jede Aminosäure zufällig ist, sondern nur jene an bestimmten, aus der Struktur abgeleiteten Positionen. Damit können neue diagnostische Marker und bisher unbekannte Korrelationen zwischen Krankheit und spezifischen Antikörperreaktivitäten gefunden werden, darunter z.B. Marker für Tumorerkrankungen, für Herz-Kreislauferkrankungen wie Herzinfarkt, für multiple Sklerose und Parkinson, für alle Arten von Autoimmunkrankheiten und Allergien und für alle Arten von Infektionskrankheiten.

Das entstehende Muster von Markern kann zum einen selbst durch die Korrelation zu bestimmten Krankheitsbildern die diagnostische Aussage ermöglichen. Die neu gefundenen Marker können aber auch gesondert auf Träger aufgebracht und bei künftigen Untersuchungen verwendet werden.

Ähnliches gilt auch für die Verwendung von sogenannten Unigeneproduct Arrays. Der Mensch besitzt ca 100.000 Gene, die durchschnittlich etwa 500 Aminosäuren codieren. In wenigen Jahren werden über 90% dieser Gene bekannt sein. Wenn man jedes dieser Genprodukte durch durchschnittlich 100 überlappende 15er Peptide abdeckt, die jeweils um 5 Aminosäuren gegeneinander verschoben sind, so werden ca. 10 Millionen verschiedene Peptide benötigt, um alle menschlichen Genprodukte abzudecken. In Fig. 17 ist ein solcher Array schematisch dargestellt.

Als Zwischenziele bieten sich dabei auch Expressionsarrays an, die jeweils nur einen Teil der menschlichen Genprodukte abdecken, z.B. "Onkogenarrays", "Immunologiearrays" etc.

Wie oben für die vollständigen Peptidbibliotheken beschrieben eignen sich diese Arrays im besonderen Maße für die Analyse des Serum-Antikörperprofils von Autoimmunpatienten (s.o.). Dabei sind besonders klare Signale zu erwarten, da hier ein Array mit einzelnen definierten und relativ langen Peptiden vorliegt.

Auch diese Arrays eignen sich im besonderen Maße für die Analyse des Serums von Patienten, die Krankheiten mit bisher unbekannten Ursachen haben. Diese Arrays könnten z.B. die Frage beantworten, ob Multiple Sklerose oder Parkinson oder verschiedene Krebskrankheiten eine Autoimmunkomponente haben. Umgekehrt betrachtet eignen sich diese Arrays damit auch in besonderem Maße als Diagnostikum für die genannten Krankheiten.

Wie oben beschrieben können die Arrays auch bei der Suche nach Interaktionspartnern eingesetzt werden und so z.B. bei der Beantwortung der Frage helfen, welche menschlichen Genprodukte die Eintrittspforte für definierte Viruspartikel darstellen.

In analoger Weise kann auch versucht werden, Krankheiten zu Bindungsmustern an andere Molekülbibliotheken, wie D-Peptidbibliotheken oder Oligosaccharidbibliotheken , zu korrelieren. Diese Methode ist dabei nicht auf menschliche Krankheiten beschränkt, sondern eignet sich zur Untersuchung forensischer und veterinärmedizinischer Fragestellungen ebenso wie zur Analyse anderer Flüssigkeiten, von Pflanzenextrakten bis hin zu Extrakten aus Mikroorganismen.

Bei einer weiteren Anwendung werden potentiell therapeutisch interessante Moleküle, wie z.B. D-Peptide, die nicht von den menschlichen Verdauungsenzymen abgebaut werden können, auf einem Träger angeordnet und anschließend mit medizinisch relevanten Molekülen, insbesondere mit Krankheitserreger-spezifischen Proteinen oder mit Mischungen von Krankheitserreger-spezifischen Proteinen in Kontakt gebracht. Dies ermöglicht die gezielte und schnelle Suche nach Bindungspartnern an diese medizinisch relevanten Moleküle. Analog ist auch die Suche nach Enzymliganden, Enzymsubstrat-Analoga oder Enzyminhibitoren möglich.

Anschließend kann dann die Bindung an die medizinisch relevanten Moleküle detektiert werden, z.B. im Wege der Biotinylisation oder der Fluoreszenz-Markierung, so dass die D-Peptide oder Aptamere identifiziert werden können, die zumindest Teile des Krankheitserregers binden. Diese D-Peptide oder Aptamere können anschließend nacheinander daraufhin getestet werden, ob sie den Krankheitserreger hemmen. Hat man z.B. ein Enzym des Krankheitserregers (z.B. Protease von HIV, reverse Transcriptase etc.) in geeigneter Menge vorliegen, so kann dieses Enzym fluoreszenzmarkiert werden (entweder direkt oder mit Hilfe eines Antikörpers, oder durch die rekombinante Expression eines kleinen Peptid-tags, das mit Hilfe eines monoklonalen Antikörpers anfärbbar ist). Damit kann festgestellt werden, an welche D-Peptide das Enzym gebunden hat. Anschließend führt man eine weitere Färbereaktion durch, die durch die Enzymaktivität verursacht wird. Beispielsweise präzipitiert die Spaltung durch die HIV-Protease ein fluoresziierendes Peptid, das man nachweisen kann. So erhält man D-Peptide, die das Enzym nicht nur binden, sondern auch gleichzeitig hemmen.

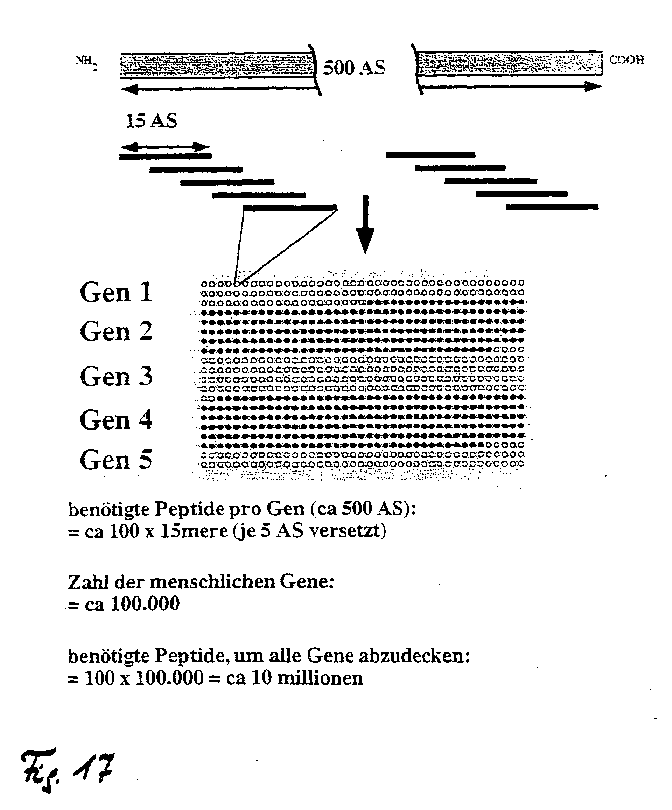

In Fig. 18 ist die vorteilhafte Verwendung solcher Arrays schematisch dargestellt. Sie ermöglichen dabei zwei verschiedene Enzymnachweise:
a. die Identifizierung von Peptiden, die das Enzym binden (Fig. 18, 44), ohne seine Enzymaktivität zu blockieren und
b. die differentielle Identifizierung von Peptiden, die das Enzym binden und gleichzeitig seine Enzymaktivität blockieren (Fig. 18, 45). Diese letzteren Module von D-Peptiden eignen sich im besonderen Maße als Bausteine für potentielle Therapeutika.

In einem nächsten Schritt können dann inhibitorisch wirkende Bindungsmodule um weitere Monomere verlängert oder miteinander kombiniert werden, gefolgt von einem weiteren Nachweis der inhibitorischen Wirkung.

Um nachzuweisen, an welchen der auf dem Träger gebundenen Moleküle sich Moleküle angelagert haben, kann das Blutserum oder die DNA nach oder vor dem In-Kontakt-Bringen des Trägers mit dem Blutserum oder der DNA mit einem mit dem Blutserum oder der DNA reagierenden, insbesondere Bindungen eingehenden Stoff in Kontakt gebracht werden. Zweckmäßigerweise wird dabei der mit dem Blutserum oder der DNA reagierende Stoff vor dem In-Kontakt-Bringen mit dem Serum oder der DNA mit einem zur Lumineszenz anregbaren Stoff, insbesondere einem durch Einstrahlung von Laserlicht zur Fluoreszenz anregbaren Farbstoff, eingefärbt. Solche Farbstoffe sind z.B. unter den Namen "Cy3", "Cy5", "FITC" oder "TRITC" im Handel erhältlich, wobei vorteilhaft bereits eine ganze Palette von Konjugaten dieser Fluoreszenz-Farbstoffe zur Verfügung steht (z.B. Ziege-Anti-Mensch-Antikörper konjugiertes Cy5).

Wird das zu untersuchende Blutserum mit einem für die Immunglobuline des Typs E spezifischen Nachweisreagenz in Kontakt gebracht, so lassen sich damit evtl. beim Patienten bestehende Allergien ermitteln, da die Immunglobuline des Typs E für die allergischen Reaktionen wie Asthma oder Heuschnupfen verantwortlich sind. Nicht-Allergiker haben praktisch keine IgEs in ihrem Blutserum, Allergiker unterschiedliche Mengen, die unterschiedliche Allergene erkennen lassen. Schließlich ermöglicht die Erfindung die Suche nach spezifischen Bindungspartnem für ein Zielmolekül aus einer hochkomplexen Bibliothekt und damit - durch die gleichzeitige Identifizierung vieler (unterschiedlich stark bindender) Bindungspartner - nach den für die Bindung des Liganden an das Zielmolekül verantwortlichen Strukturparametern. Dadurch wird der Weg zu Leitstrukturen stark vereinfacht. Beispielsweise können dazu mit den oben genannten Methoden erhaltene Signalmuster automatisch mit Strukturparametern oder Strukturmodellen der identifizierten Liganden aus der benützten Bibliothek korreliert werden.

Die genannten vorteilhaften Anwendungen sind dabei keineswegs auf Peptid-Arrays und die Untersuchung von Blutserum beschränkt. Daneben gibt es eine Fülle von weiteren Anwendungsmöglichkeiten, wann immer vorteilhaft eine große Zahl von potenziellen Bindungspartnern auf Bindung untersucht werden soll. Insbesondere gilt das für die Kombination von 2 Molekülbibliotheken, wovon die eine Bibliothek die genannten Arrays darstellen und das genannte Blutserum nur ein Beispiel unter vielen für eine solche zweite Bibliothek ist. Die Identifizierung der Bindungspartner erfolgt bei den genannten Arrays dabei aufgrund ihrer Position auf dem Array, während für die Identifizierung des zweiten Bindungspartners noch weitere vorteilhafte Nachweismethoden, wie z.B. die Massenspektrometrie zur Anwendung kommen können.

Nachfolgend sind einige Beispiele der Durchführung erfindungsgemäßer Verfahren bzw. der Anwendung erfindungsgemäßer Vorrichtungen unter Bezugnamen auf die Zeichnung beschrieben.

Es zeigen:
- Figur 1:: Kombinatorische Synthese:
I. für die kombinatorische Synthese geeignete Substanzen (2) werden auf einen Träger (1) aufgebracht
II. die unterschiedlichen Substanzen (2) werden an den Träger (1) gekoppelt (11)
III. die nicht gekoppelten Substanzen (2) werden weggewaschen
IV. die Schutzgruppen (14) werden abgespalten
V. erneut werden Substanzen (2) auf den Träger (1) aufgebracht und der nächste Zyklus beginnt.
- Figur 2:: Das konfokale Lasermikroskop als Ausleseprinzip für Arrays Wenn Arrays mit Hilfe eines konfoklaen Lasermikroskops ausgewertet werden, müssen alle 3 Dimensionen des Raums durchsucht werden, bis das gewünschte Signal eindeutig identifiziert werden kann. Verglichen damit ist z.B. ein Scanner viel schneller:
1. Er hat nicht nur einen Laser zur Verfügung sondern arbeitet parallel mit einem eindimensionalen Array von Leuchtdioden.
2. Da das Licht der Leuchtdioden nahezu parallel den Träger durchstrahlt, fällt beim Scanner das suchen in der dritten Dimension des Raumes weg.
- Figur 3:: Lithographische Verfahren für die kombinatorische Synthese.
Bei den gebräuchlichen lithographischen Syntheseseverfahren wird aufgrund der Einwirkung von Licht der Syntheseort für die aktivierten Monomere zugänglich gemacht (Fig. 3, I.), oder aber es werden lichtempfindliche Schutzgruppen abgespalten (Fig. 3, II.), wodurch eine lokal definierbare Kettenverlängerung ermöglicht wird. Ein Vorteil beider Verfahren ist ihre hohe Auflösung. Ein Nachteil beider Verfahren ist, daß nacheinander der gesamte Synthesezyklus für jedes einzelnes Monomer durchlaufen wird, was auf Kosten der Qualität geht.
Ia. Nach dem einwirken von Licht (16) kann eine lichtempfindliche Schutzschicht (17) orstgenau definiert (18) entfernt werden.
Ib. Dadurch können aufgebrachte Monomere (2) für die kombinatorische Synthese ortsgenau definiert (18) an den Träger (1) koppeln (11).
IIa. Nach dem Einwirken von Licht (16) werden lichtempfindliche Schutzgruppen (15) orstgenau definiert (18) abgespalten.
IIb. Dadurch können aufgebrachte Monomere (2) für die kombinatorische Synthese ortsgenau definiert (18) an den Träger (1) koppeln (11).
- Figur 4:: Nachteile der lithographischen Syntheseverfahren
Bei allen Druckverfahren wird immer eine ganze Schicht von unterschiedlichen Monomeren (2) auf den Träger (1) aufgebracht, die dann alle zusammen einen Kopplungszyklus durchlaufen bevor wiederum die nächste Schicht von Monomeren (2) aufgedruckt wird. Bei den in Fig. 3 dargestellten lithographischen Syntheseverfahren dagegen muß jede Art von Monomer (2) nacheinander einzeln aufgebracht, gekoppelt (11) und überschüssige Monomere weggewaschen werden. Dies bedeutet, daß für die Synthese desselben Arrays von Oligomeren, die lithographischen Synthesemethoden N x mehr Kopplungszyklen durchlaufen müssen als bei den Druckverfahren nötig. Die Zahl N steht dabei für die Anzahl unterschiedlicher Monomere (2), d.h. für die Synthese eines Arrays von Oligopeptiden brauchen die lithographischen Synthesen 20x mehr Kopplungszyklen verglichen mit den Druckverfahren.
- Figur 5:: Druckverfahren für die kombinatorische Synthese.
Ia. Tonerpartikel, bzw. Transporteinheiten (19), die für eine kombinatorische Synthese geeignete Substanzen (2) enthalten, werden ortsgenau definiert (18) auf einen geeigneten derivatisierten Träger (1) aufgebracht.
Ib. Anschließend werden die Monomere (2) für die kombinatorische Synthese aus den Tonerpartikeln, bzw. aus den Transporteinheiten (19) freigesetzt und koppeln (11) dann ortsgenau definiert (18) an den Träger (1).
IIa. Flüssigkeiten, die für eine kombinatorische Synthese geeignete Substanzen (2) enthalten, werden ortsgenau definiert (18) auf einen geeigneten derivatisierten Träger (1) aufgebracht.
IIb. Anschließend koppeln (11) die Monomere (2) für die kombinatorische Synthese dann ortsgenau definiert (18) an den Träger (1).
IIa. & IIb beschreiben die im Stand der Technik verwendeten Druckverfahren für die kombinatorische Synthese. Ia. & Ib beschreiben das erfindungsmäßige neue Verfahren. Es kombiniert die Vorteile der Druckmethoden mit den Vorteilen der lithographischen Methoden. Auch hier wird wie bei den lithographischen Verfahren die hohe Auflösung von Laserlicht ausgenützt, um eine dicht gepackte Molekülbibliothek zu erhalten. Andererseits kann damit aber wie bei den anderen Druckverfahren auch parallel eine ganze Schicht von unterschiedlichen Monomeren auf den Träger aufgebracht werden.
- Figur 6:: Nachteile des Spottens von Monomeren
Wenn Papier mit Farbe bedruckt wird, so dürfen die gedruckten Chromophoren möglichst wenig diffundieren, da dies die Brillianz des gedruckten Bildes stören würde. Erreicht wird dies dadurch, daß aufgrund von schnellflüchtigen Bestandteilen in den verwendeten Tonerflüssigkeiten die aufgebrachten Chromophoren sehr schnell an Ort und Stelle fixiert werden. Darüberhinaus sind die verwendeten Chromophoren rel. groß, was ihre Diffusionsrate erheblich einschränkt. Außerdem werden spezielle, stark saugfähige Hochglanzpapiere verwendet.
Die aufgebrachten Monomere (für eine kombinatorische Synthese) werden dagegen sehr viel weiter diffundieren, da das für die Synthese verwendete Lösungsmittel sehr schlecht flüchtig ist, da Zeit für die Kopplung der Reaktionspartner gebraucht wird. Die verwendeten Monomere sind darüberhinaus vergleichsweise klein, was ihre Diffusionsrate erheblich erhöht. Spezialpapiere eignen sich normalerweise nicht als Träger einer Molekülbibliothek.
- Figur 7:: Schematischer Vergleich der Partikel eines normalen Toners (46, 47, 48) mit einem "Aminosäurentoner" (46, 49, 2, 50):
- das Äquivalent der bei Raumtemperatur festen Polystyrolkügelchen (47) des normalen Toners ist in diesem Fall Diphenylformamid (50) mit einem Schmelzpunkt bei etwa 71 °C.
- der magnetische Anteil (46) wird in beiden Fällen von Magnetitpartikeln beigesteuert.
- anstelle der Chromophoren enthält der "Aminosäurentoner" die mit einer N-terminalen Schutzgruppe versehenen aktivierten Aminosäuren (49, 2).
- Figur 8: Funktionsweise eines Laserdruckers:
Tonerpartikel (19), bestehend aus kleinen Polystyrolkügelchen mit einem magnetischen Anteil haften aufgrund des magnetischen Anteils an einer Magnetwalze (21). Dort werden sie elektrostatisch aufgeladen und springen aufgrund dieser Ladung auf ortsgenau definierte Bereiche einer Laser-beschreibbaren Walze (22). Die Definition der Bereiche übernimmt der Laser, der bestimmte Bereiche durch ein und ausschalten "beschreibt". Aufgrund elektrischer Anziehungskräfte springen (23) die Tonerpartikel (19) von der Magnetwalze (21) auf die vom Laser beschriebenen Bereiche der Walze (22). Von dort springen die Tonerpartikel (19) auf den Träger (24) (z.B. Papier oder eine Kopierfolie) und werden durch eine heiße Walze (25) eingeschmolzen, bzw. im Falle von Aminosäuren-Tonerpartikeln werden die darin eingeschlossenen Substanzen beweglich gemacht (26).
- Figur 9: Funktionsweise eines Farblaserdruckers:
Wie beim schwarz-weiß Laserdrucker (Fig. 8) bestehen beim Farblaserdrucker die Tonerpartikel (20) aus kleinen Polystyrolkügelchen mit einem magnetischen Anteil. Sie haften aufgrund des magnetischen Anteils an einer Magnetwalze (21). Dort werden sie elektrostatisch aufgeladen und springen aufgrund dieser Ladung auf ortsgenau definierte Bereiche einer Laser-beschreibbaren Walze (22). Die Definition der Bereiche übernimmt der Laser, der bestimmte Bereiche durch ein und ausschalten "beschreibt". Aufgrund elektrischer Anziehungskräfte springen (23) die Tonerpartikel (20) von der Magnetwalze (21) auf die vom Laser beschriebenen Bereiche der Walze (22). Von dort springen die Tonerpartikel (20) auf den Träger (24) (z.B. Papier oder eine Kopierfolie) und werden durch eine heiße Walze (25) eingeschmolzen, bzw. im Falle von Aminosäuren-Tonerpartikeln werden die darin eingeschlossenen Substanzen beweglich gemacht (26).
Im Unterschied zum schwarz-weiß Laserdrucker (Fig. 8), muß ein Farblaserdrucker jedoch nicht nur einen Toner (20) aufdrucken, sondern 4 verschiedenfarbige (31, 32, 33, 34) Tonerpartikel (20) exakt nebeneinander drucken.
In Fig. 9 wird schematisch ein Weg gezeigt, wie dieses Problem gelöst wird:
- die Laser-beschreibbare Walze (22) ist wesentlich größer als beim schwarz-weiß Laserdrucker (Fig. 8)
- damit kann der Laser ein ganzes Blatt auf diese Walze "schreiben"
- der Laser "beschreibt" die Laser-beschreibbaren Walze (22) mit der ersten Farbe
- eine Magnetwalze (21), die den ersten Farbtoner (21, 31) transportiert fährt an die beschreibbare Walze (22) heran
- von dort springen die Tonerpartikel (21, 31) auf die "beschriebene" beschreibbare Walze (22)
- die beschreibbare Walze (22) ist eng mechanisch mit einer genauso großen zweiten Walze (35) gekoppelt
- auf diese Walze (35) wird ein Träger (1) aufgezogen und dort fixiert
- die beiden Walzen (22 & 35) drehen sich gegeneinander
- damit wird der erste Toner (27) auf den Träger (1) übertragen, der an der Walze (35) fixiert bleibt
- der Laser "beschreibt" die beschreibbare Walze (22) mit der zweiten Farbe
- anschließend fahren weitere Wechselmagnetwalzen (21, 32, 33, 34) an die beschreibbare Walze (22) heran
- die die weiteren Farben (28, 29, 30) auf die beschreibbare Walze (22) übertragen
- von dort werden die weiteren Farben (28, 29, 30) auf den Träger (1) übertragen
- erst wenn der gesamte Druckvorgang abgeschlossen ist, wird der Träger freigegeben
Anstelle der Trägerwalze (35) kann sich auch eine sogenannte Transfereinheit befinden, die in einem Rückkopplungsmechanismus immer wieder in Bezug auf die beschreibbare Walze justiert wird.
- Figur 10: Handelsübliche Laserdrucker haben eine Auflösung von 600dpi, d.h. die einzelnen Punkte, die diese Geräte drucken, haben einen Durchmesser von ca 40µm.
- Figur 11: Ein Druckbild eines handelsüblichen Laserdruckers mit 600dpi Auflösung wurde mit einem handelsüblichen Scanner mit 600dpi Auflösung abgerastert. Dabei zeigt das Abrastern des eingescannten Laserdruckbildes bei hoher Vergrößerung typischerweise keinen einzigen falsch gesetzten Pixel.
- Figur 12: Komplexität kombinatorischer Peptidbibliotheken
- Figur 13: Gerät für die kombinatorische Synthese.
Im Unterschied zu dem in Fig. 9 beschriebenen Farblaserdrucker ist bei diesem Gerät die heiße Walze (25) durch eine Vorrichtung (36) ersetzt worden, die es ermöglicht Kopplungsreagentien oder Waschlösungen in gasförmigem oder in flüssigem Zustand in Kontakt mit der sich drehenden Trägerwalze (35) zu bringen. Gasförmige Substanzen (auch heiße Luft) werden mit Hilfe einer breiten Düse zugeführt (36), während Flüssigkeiten z.B. durch ein Endlosband (36) in Kontakt mit dem auf der Trägerwalze (35) fixierten Träger (1) der Molekülbibliothek gebracht werden. Für das beweglich machen der Substanzen (2) kann auch eine Zeile aus Infrarotlichtquellen (36) verwendet werden. Die Wechselmagnetwalzen (21) mit den 4 verschiedenfarbigen Tonern (31, 32, 33, 34) wird ausgetauscht gegen Wechselmagnetwalzen (21) mit den 20 verschiedenen Aminosäurentonern (2, 19).
- Figur 14: Gerät für die kombinatorische Synthese mit eingebautem Rückkopplungsmechanismus.
Eine Scannereinheit (37) rastert ein auf den Träger (1) oder die Trägerwalze (35) aufgebrachtes Muster ab (38) und vergleicht es mit demselben, bereits gespeicherten Muster (39). Wird dabei eine Abweichung vom Sollwert festgestellt, so wird das Bild, das in den Druckerspeicher geladen wird, elektronisch um diese Abweichung verschoben (40). Damit wird erreicht, dass die Transporteinheiten (19) bzw. die darin eingeschlossenen Substanzen (2) wiederholbar ortsgenau neben oder übereinander gedruckt werden können.
- Figur 15: Vollständige Peptidbibliotheken
Arrays der dargestellten Decamergemische können mit Hilfe des weiter oben beschriebenen Verfahrens auf einem Träger synthetisiert werden. Dabei werden auf dem Array alle möglichen Kombinationen der mit N bezeichneten Aminosäuren abgedeckt.
- Figur 16: Korrelation von Krankheit mit Peptidmuster
Die differentielle Färbung eines hochkomplexen Arrays von Peptiden mit Kontrollserum und dem Serum von Patienten ergibt einerseits Peptide (und damit Peptidsequenzen), die von beiden Seren erkannt werden (42) und andererseits Peptide, die spezifisch von Patientenseren erkannt werden (43). Dies ermöglicht die Identifizierung von Patientenspezifischen Färbemustern. Im angegebenen Beispiel werden Peptide identifiziert (43), die exprimierten Genprodukten von Helicobacter pylori entsprechen, dem Verursacher von Magengeschwüren.
Dieses Verfahren ist nicht auf eine Krankheit beschränkt, es können damit auch mehrere Krankheiten parallel diagnostiziert werden, oder umgekehrt mit Hilfe der aufgefundenen Peptide bisher unbekannten Krankheitsursachen nachgespürt werden.
- Figur 17: Array von Genprodukten, Unigeneproduct-Arrays
Die ca 100.000 Gene des Menschen (d.h. sein Genom) codieren durchschnittlich etwas weniger als 500 Aminosäuren pro Gen. In wenigen Jahren werden die meisten dieser Gene bekannt sein. Jedes der entsprechenden Genprodukte kann durch durchschnittlich 100 überlappende 15er Peptide repräsentiert werden, die jeweils um 5 AS gegeneinander verschoben sind. Insgesamt werden dann ca 10 millionen verschiedene Peptide benötigt, um alle 100.000 menschliche Genprodukte (d.h. sein Proteom) abzudecken.
- Figur 18: Suche nach Enzyminhibitoren
Zwei verschiedene Enzymnachweise ermöglichen:
a. die Identifizierung von Peptiden, die das Enzym binden (44), ohne seine Enzymaktivität zu blockieren.
b. die differentielle Identifizierung von Peptiden, die das Enzym binden und gleichzeitig seine Enzymaktivität blockieren (45). Diese letzteren Module von D-Peptiden eignen sich als Bausteine für potentielle
Therapeutika.

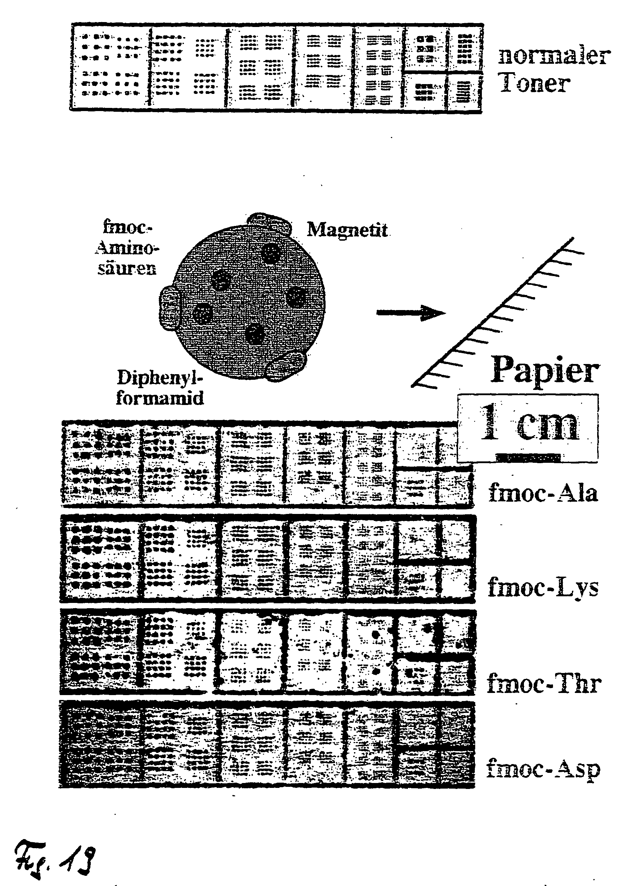

- Figur 19: Vergleich der Druckqualität von normalem käuflichem Toner mit verschiedenen "Aminosäurentonern":
Die verschiedenen Toner wurden in eine Tonerkassette gefüllt und mit einem Laserdrucker auf normales Papier gedruckt. Die Färbung der "Aminosäurentoner" kommt durch die darin enthaltenen Magnetitpartikel zustande.

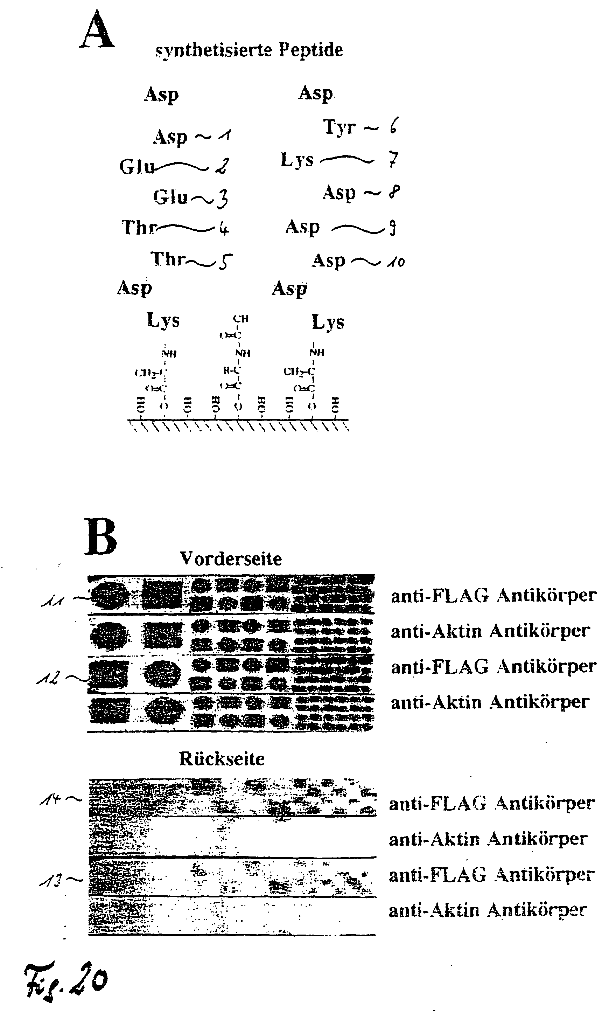

- Figur 20: An die freien Aminogruppen von derivatisiertem Papier wird uniform nacheinander Lysin gefolgt von Asparaginsäure gekoppelt. Anschließend werden schachbrettartig zwei verschiedene Peptide auf einem Träger aus derivatisiertem Papier synthetisiert. Dabei werden die den in Fig. 20 dargestellten Aminosäuren 1 bis 10 entsprechenden "Aminosäurentoner" hergestellt. Die in Fig. 20 markierten Aminosäuren 1 bis 5 werden in einem regelmäßigen Muster von Ovalen 11 aufgedruckt, während die markierten Aminosäuren 6 bis 9 als zweites Muster aus regelmäßig angeordneten Rechtecken 12 gedruckt werden. Beide Muster greifen dabei schachbrettartig ineinander. Die N-terminale Aminosäure Asparaginsäure wird anschließend in einem letzten Schritt wieder uniform an den Träger gekoppelt, gefolgt vom Abspalten der Schutzgruppen.
Die Syntheseorte der bei (A) dargestellten Peptide korrespondieren zu den bei (B) sichtbaren grauen Ovalen bzw. Rechtecken. Die Papierstreifen werden mit Milchpulver in PBS geblockt und mit anti FLAG M1 Antikörper bzw. mit einem anti Aktin Antikörper inkubiert. Der Nachweis des gebundenen 1. Antikörpers erfolgt mit Peroxidasekonjugiertem Ziege anti Maus Antikörper (Substrat 13) bzw. mit alkalischer Phosphatase konjugiertem Ziege anti Maus Antikörper (Substrat 14).
- Figur 21: Kopplung der Nucleoside mit DMTr-Schutzgruppe am 5'-OH-Ende an die auf einem festen Untergrund verankerten NH2-Gruppen.

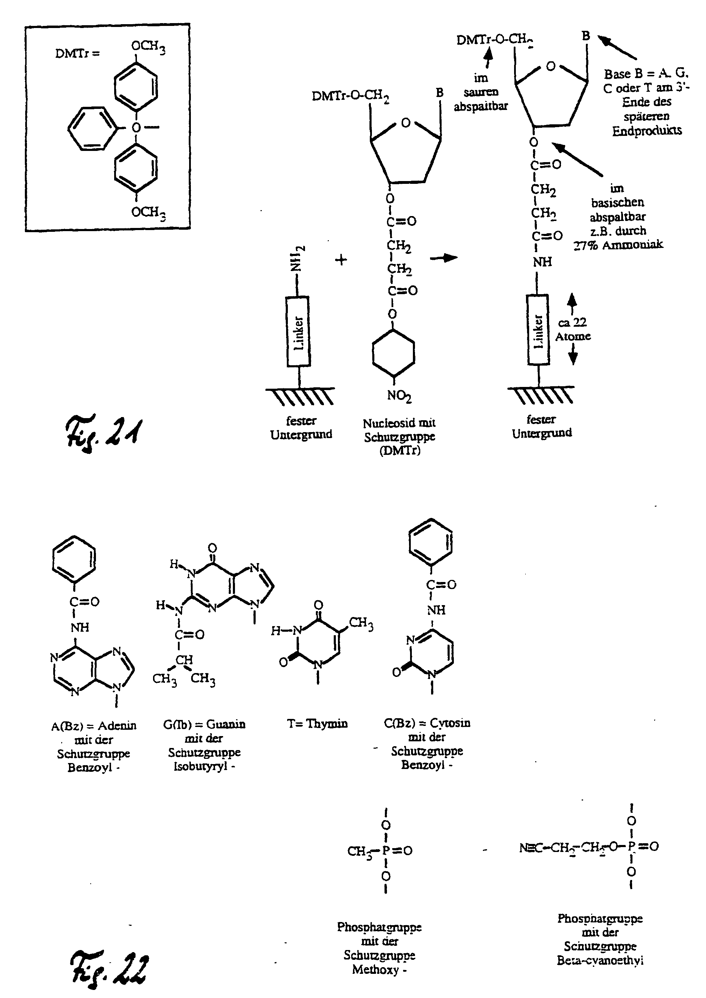

- Figur 22: Gebräuchliche Schutzgruppen (für die Basen und für die Phosphatgruppen) bei der Oligosynthese.

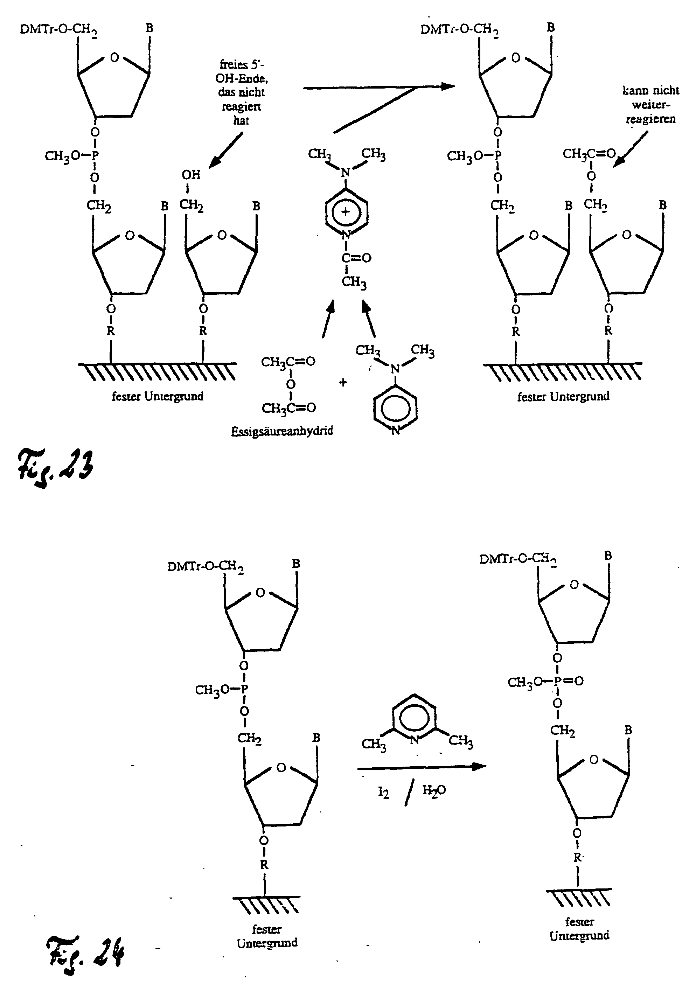

- Figur 23: "Capping" von nicht-reaktiven 5'OH-Enden.

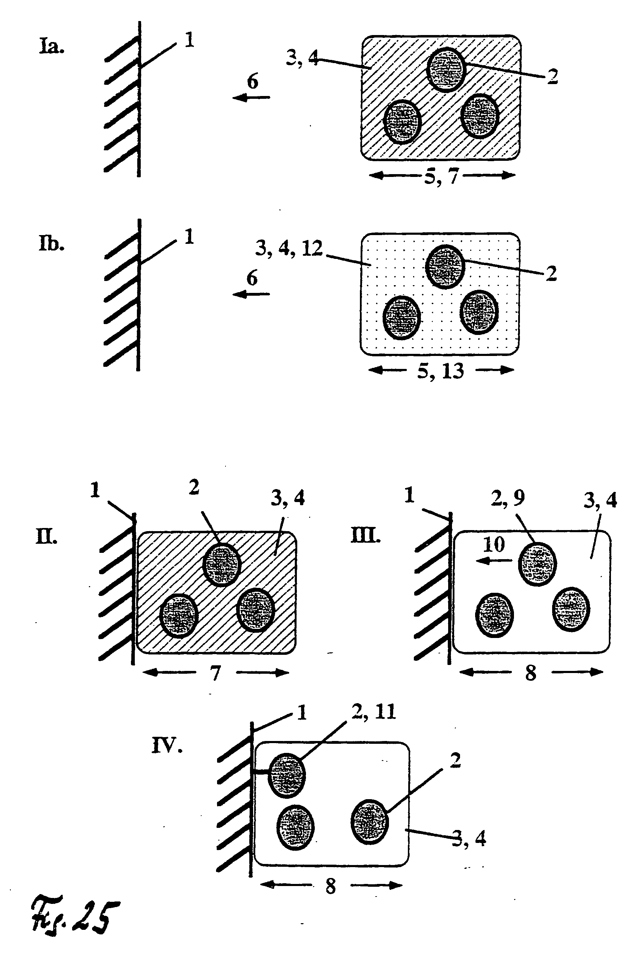

- Figur 24: Oxidation von trivalenten Phosphatgruppen.
- Figur 25: Schemaskizze des Verfahrens gemäß Anspruch 1.

### a) Umbau eines Farblaserdruckers in eine Oligomer-Synthesemaschine (Fig. 13)

Die Programmsteuerung eines handelsüblichen Farblaserdruckers wird dahingehend geändert, dass:
- Der an die Trägerwalze (oder an die Transferwalze) gekoppelte Träger (insbesondere für die Peptidsynthese oder die Oligonukleotidsynthese derivatisierte Kopierfolie bestehend im wesentlichen aus Polystyrol oder für die Peptidsynthese oder die Oligonukleotidsynthese derivatisiertes Papier) auf der Trägerwalze (oder auf der Transferwalze) fixiert bleibt, bis ein externes Steuersignal diese Fixierung an die Trägerwalze beendet
- anstelle von 4 verschiedenen Magnet-Wechselwalzen mit angehängten Tonerbehältern 24 verschiedene Magnet-Wechselwalzen mit angehängten Tonerbehältern in die Nähe der beschreibbaren Walze dirigieren.

Alternativ kann die Transfereinheit auch nacheinander in 6 umgebaute Farblaserdrucker eingesetzt werden, die jeweils 4 unterschiedliche Tonerbehälter enthalten. Insgesamt werden so bis zu 24 unterschiedliche Tonerpartikel in einer Schicht auf die Transfereinheit aufgebracht. Die je nach Gerät unterschiedliche Positionierung des Lasers wird vorher vermessen und bei der Druckvorlage berücksichtigt.

Des weiteren wird ein externes Gerät gebaut, bestehend aus
- einer Heizeinheit, insbesondere durch Infrarotlicht oder durch einen Heißluftfön
- einer drehbaren Walze mit der zeitlich programmierbar verschiedene Flüssigkeiten auf den auf der Trägerwalze oder der Transfereinheit fixierten Träger zugeführt werden können
- einer drehbaren Walze mit der zeitlich programmierbar verschiedene Flüssigkeiten von auf dem auf der Trägerwalze fixierten Träger abgeleitet werden können.

### b) Umbau eines weiteren Farblaserdruckers in eine Oligomer-Synthesemaschine (Fig. 14)

An das Endlosband einer Transferwalze eines handelsüblichen Farblaserdruckers wird ein Träger fixiert. Auf dem Träger, insbesondere aber auf der Transfereinheit sind von einer opto-elektronischen Abtasteinheit erkennbare Strukturen angebracht, die es erlauben, in einem Rückkopllungsmechanismus die Position der Transfereinheit und damit die Position des darauf fixierten Trägers relativ zur Position des für den Transfer der Tonerpartikel verantwortlichen Lasers zu justieren.

Des weiteren wird ein weiteres externes Gerät gebaut, bestehend aus
- einer Heizeinheit, insbesondere durch Infrarotlicht oder durch einen Heißluftfön
- einer Wanne, mit der dem auf der Transfereinheit fixierten Träger zeitlich programmierbar verschiedene Flüssigkeiten zugeführt werden können
- einer drehbaren Walze mit der zeitlich programmierbar verschiedene Flüssigkeiten von auf dem auf der Trägerwalze fixierten Träger abgeleitet werden können.

Die genannte Transfereinheit mit daran fixiertem Träger kann zwischen einzelnen Kopplungszyklen aus dem genannten weiteren Farblaserdrucker herausgenommen werden und in das genannte weitere externe Gerät eingespannt werden. Nach der erfolgten Zufuhr und Abfuhr diverser Flüssigkeiten kann die genannte Transfereinheit aufgrund der genannten opto-elektronischen Abtasteinheit wieder genau relativ zur Position des für den Transfer der Tonerpartikel verantwortlichen Lasers justiert werden.

### c) Herstellung von Aminosäurentonern

Mit Schutzgruppen, insbesondere mit fmoc Schutzgruppen versehene einzelne Aminosäuren, insbesondere auch die entsprechenden Anhydride werden zusammen mit Magnetitpartikeln bei 75°C in Diphenylformamid gelöst, schockgefroren und kleingemahlen, sodass möglichst uniforme Partikel von ca 1-200 µm Durchmesser, insbesondere von 5-40 µm Durchmesser entstehen. Diese Partikel werden in Tonerkassetten gefüllt und auf Papier gedruckt. Fig. 19 vergleicht die Druckqualität eines normalen Toners mit diversen Aminosäurentonern.

### d) Herstellung von Phosphoramidit-Tonerpartikeln

Mit Schutzgruppe versehene einzelne Phosphoramidite werden zusammen mit Magnetitpartikeln bei 25°C in Diphenylformamid / Acetonitril gelöst, schockgefroren und der lösliche Bestandteil bei tiefen Temperaturen sublimiert. Anschließend werden sie kleingemahlen, sodass möglichst uniforme Partikel von ca 1-200 µm Durchmesser, insbesondere von 2-40 µm Durchmesser entstehen. Diese Partikel werden in Tonerkassetten gefüllt und auf Papier gedruckt.

### e) Synthese eines Peptidarrays mit Hilfe eines Laserdruckers

Die in Beispiel (c) beschriebenen Aminosäuretoner (siehe auch Fig. 19) werden zur Synthese von zwei schachbrettartig ineinander greifenden Peptidmustern eingesetzt.

Zunächst wird Papier mit Standardmethoden mit freien Aminogruppen derivatisiert. Anschließend werden mit Standardmethoden zwei weitere Aminosäuren uniform an das Papier gekoppelt. Mit den bei Beispiel (c) beschriebenen individuellen Aminosäurentonern wird anschließend ein schachbrettartiges Muster von Ovalen und anschließend nach dem wechseln der Tonerkassette ein damit ineinandergreifendes Muster von Rechtecken auf das derivatisierte Papier gedruckt. Insgesamt werden so 5 Schichten von Aminosäuren aufgedruckt, jeweils gefolgt von dem Koppeln der in den Aminosäurentonern enthaltenen mit Schutzgruppen versehenen Aminosäuren und vom Abspalten der N-terminalen Schutzgruppe. In einem weiteren Schritt wird wiederum uniform mit Standardmethoden eine letzte N-terminale Aminosäure gekoppelt, gefolgt von dem Abspalten der Schutzgruppen an den Seitenketten der Peptidkette mit Standardmethoden. Derart werden 2 schachbrettartig ineinander greifende Muster der Peptide (N-Terminus) - DYKDDDDK - (Träger) und (N-Terminus) - DDEETTDK - (Träger) synthetisiert.

### f) Nachweis eines mit Hilfe eines Laserdruckers synthetisierten Peptidmusters mit Standardmethoden

Das in Beispiel (e) beschriebene derivatisierte Papier, an das zwei schachbrettartig ineinander greifende Peptidmuster gekoppelt wurden, wird in kleinere Stücke zerschnitten und zunächst werden unspezifische Bindungen mit einer geeigneten wäßrigen Lösung, wie zum Beispiel 2 % Milchpulver in PBS abgeblockt. Im gleichen Puffer werden zwei verschiedene monoklonale Antikörper (aus der Maus) verdünnt. Danach werden einzelne Papierstücke unter leichtem Schütteln für 60 Minuten mit dem einen bzw. mit dem anderen monoklonalen Antikörper gefärbt und anschließend drei Mal gewaschen. Der an ein Enzym gekoppelte Ziege-Anti-Maus-Antikörper wird in 2% Milchpulver in PBS verdünnt; anschließend wird damit der Träger unter leichtem Schütteln für 60 Minuten benetzt und sodann drei Mal gewaschen. Fig. 20 zeigt die anschließende Färbung der Papierstücke mit verschiedenen Enzymsubstraten: Der monoklonale Mausantikörper FLAG M1 (Sigma), erkennt spezifisch das Peptid (N-Terminus) - DYKDDDDK - (Träger), bzw. das mit Hilfe eines Laserdruckers generierte schachbrettartige Peptidmuster aus Rechtecken.

### g) Synthese einer vollständigen 5mer Peptidbibliothek mit Hilfe eines abgewandelten Farblaserdruckers

Ein geeigneter flacher Träger wird mit Standardmethoden mit freien Aminogruppen derivatisiert. Dabei eignen sich insbesondere Papier oder Kopierfolien, die im wesentlichen aus Polystyrol bestehen. An die freien Aminogruppen des Trägers wird mit Hilfe von dem Fachmann geläufiger Standard fMoc-Peptidsynthese unter wasserfreien Bedingungen zunächst ein geeigneter Spacer, insbesondere von 2-3 Aminosäuren Länge synthetisiert. Optional können diesem Spacer mit Standardmethoden 2 oder 3 weitere Schichten von gekoppelten Aminosäuren hinzugefügt werden, vorzugsweise von Aminosäurengemischen aus 19 oder 20 verschiedenen Aminosäuren (d.h., optional ohne Cystein). Anschließend wird der derivatisierte Träger auf der Trägerwalze oder der Transfereinheit des in Beispiel (a) oder des in Beispiel (b) beschriebenen umgebauten Farblaserdruckers befestigt.

Die Tonerbehälter des in Beispiel (a) oder des in (b) beschriebenen umgebauten Farblaserdruckers enthalten verschiedene der in Beispiel (c) beschriebenen Aminosäurentoner.

Anschließend wird der Druckvorgang gestartet, sodass im wesentlichen nach dem Funktionsprinzip eines normalen Farblaserdruckers, insbesondere 19 oder 20 verschiedene Aminosäurentoner ortsgenau nebeneinander gedruckt werden. Dabei wird der Träger in 19 oder 20 voneinander getrennte, ortsgenau definierte Bereiche unterteilt. Die Kopplung der ortsgenau aufgedruckten aktivierten Aminosäuren, insbesondere von Aminosäurenanhydriden findet anschließend bei etwa 65°C für 5-30 Minuten statt. Während dieses Vorgangs dreht sich die Trägerwalze (oder die Transferwalze) mit dem darauf fest fixierten derivatisierten Träger gleichmäßig unter einer Zeile von Infrarotlampen, die als Heizeinheit in Beispiel (a) oder in Beispiel (b) beschrieben wurde.

Anschließend wird der nicht umgesetzte Aminosäurentoner wie in Beispiel (a) oder in Beispiel (b) beschrieben weggewaschen, die fMoc-Schutzgruppe mit Hilfe von Standardmethoden abgespalten, erneut gewaschen und anschließend der Träger mit Hilfe der in Beispiel (a) oder in Beispiel (b) beschriebenen Heizeinheit getrocknet. Während dieser Zeit bleibt der Träger auf der Trägerwalze (oder der Transferwalze) fest fixiert, die sich während der ganzen Zeit gleichmäßig dreht.

Anschließend wird der Druckvorgang erneut gestartet, sodass wiederum 19 oder 20 verschiedene Aminosäurentoner ortsgenau neben bzw. übereinander gedruckt werden. Damit wird der Träger diesmal vorzugsweise in 19² bzw. 20² ortsgenau definierte Bereiche unterteilt. Wie gerade beschrieben, werden die aktivierten Aminosäuren an den Träger gekoppelt, der nicht umgesetzte Aminosäurentoner weggewaschen und die fmoc Schutzgruppen abgespalten.

Drei weitere analoge Druckvorgänge unterteilen den Träger damit vorzugsweise in 19⁵ bzw. 20⁵ ortsgenau definierte Bereiche. Optional können an die freien Aminotermini dieses Peptidarrays mit Standardmethoden 2 oder 3 weitere Schichten von gekoppelten Aminosäuren hinzugefügt werden, vorzugsweise von Aminosäurengemischen aus 19 oder 20 verschiedenen Aminosäuren (d.h., optional ohne Cystein).

Schließlich werden alle Schutzgruppen, auch die der Seitenketten mit 10% Silan in konzentrierter Trifluoressigsäure abgespalten, der Träger mit DMF und Methanol gewaschen und getrocknet, sodass im Endeffekt ein Träger mit beispielsweise 20⁵ = 3.200.000 verschiedenen Bereichen entsteht, die jeweils eines von allen möglichen natürlich vorkommenden C-terminal gekoppelten Pentapeptiden repräsentieren.

### h) Synthese einer vollständigen 12mer Oligonukleotidbibliothek auf einem Träger mit Hilfe eines umgebauten Farblaserdruckers

Wie in Beispiel (d) beschrieben, werden 4 verschiedene Phosphoramidit-Tonerpartikel hergestellt, die vorzugsweise 4 unterschiedliche aktivierte Monomere für die Oligonukleotidsynthese enthalten. Diese Toner werden in Tonerbehältnisse gefüllt und wie in Beispiel (a) oder (b) beschrieben mit einem umgebauten Farblaserdrucker auf einen Träger aufgedruckt.

Wie in Beispiel (h) für die Synthese einer vollständigen 5mer Peptidbibliothek beschrieben, wird dabei ein geeigneter Träger mit freien Aminogruppen (oder Hydroxylgruppen) verwendet, der mit Standardmethoden hergestellt wird. Dabei wird, falls nicht schon durch den ersten Schritt vorhanden, an die freien Aminogruppen (oder Hydroxylgruppen) mit Hilfe von dem Fachmann geläufiger Standardsynthese unter wasserfreien Bedingungen ein geeigneter Linker synthetisiert, der wiederum freie Aminogruppen (oder Hydroxylgruppen) auf dem Träger verankert, die diesmal jedoch ca. 22 Atome von der Oberfläche entfernt sind.

Wie in Beispiel (g) für die Synthese einer vollständigen 5mer Peptidbibliothek beschrieben, werden die in den 4 verschiedenen Phosphoramidit-Tonerpartikeln befindlichen Monomere durch die Heizeinheit beweglich gemacht, nachdem sie mit Tetrazol aktiviert worden sind. Anschließend koppeln sie während 2-10 Minuten an den Träger.

Die Kopplung der aktivierten Phosphoramidite (mit Schutzgruppen) an den festen Träger, das Abspalten der Schutzgruppen und die Waschschritte finden dabei unter den dem Fachmann bekannten Standardbedingungen für die Oligonukleotidsynthese statt.

Wie in Beispiel (g) beschrieben wird der nicht umgesetzte Phosphoramidit-Toner wie in Beispiel (a) oder (b) beschrieben weggewaschen, die DMTr-Schutzgruppe von dem 5'-Ende der Phosphoramidite mit Hilfe von Standardmethoden abgespalten, erneut gewaschen und anschließend der Träger mit Hilfe der in Beispiel (a) oder (b) beschriebenen Heizeinheit getrocknet. Während dieser Zeit bleibt der Träger auf der Trägerwalze fest fixiert.

Beispiele für die dabei verwendeten Schutzgruppen sind:
- 4',4'-Dimethoxytritylchlorid (DMTr) für das 5'-Ende der Phosphoramidite (Fig. 21)
- Benzoyl im Falle der Basen Adenin und Cytosin (Fig. 22)
- Isobutyryl im Falle der Base Guanin (Fig. 22)
- Methoxy - oder Beta-cyanoethyl - im Falle der Phosphatgruppen (Fig. 22)

Nach der Kopplung der Monomere an den Träger werden eventuell verbliebene freie 5'-OH-Enden dabei bei jedem Schritt mit einem "Cap" versehen, sodass sie an späteren Reaktionen nicht mehr teilnehmen können (Fig. 23). Ein letzter Schritt, in dem trivalente Phosphatgruppen oxidiert werden beschließt den Synthesezyklus (Fig. 24)

Nach der Abspaltung der DMTr-Schutzgruppe von dem 5'-Ende der Phosphoramidite wird im nächsten Schritt wie in Beispiel (g) beschrieben der Träger erneut bedruckt, sodass der Träger diesmal in vorzugsweise 4² voneinander getrennte Bereiche unterteilt wird. In jedem dieser voneinander abgegrenzten Bereiche befindet sich jeweils eines von 16 möglichen über das 3'-Ende durch einen Spacer an den Träger gekoppelten Dinukleotide deren 5'-Ende eine freie OH-Gruppe trägt.

Dieser Vorgang wird insgesamt 10x mit beispielsweise jeweils allen 4 aktivierten Phosphoramiditen wiederholt, sodass die oben beschriebenen 16 voneinander abgegrenzten Bereiche danach in insgesamt 4¹² ortsgenau definierte Bereiche unterteilt werden, bei jedem Syntheseschritt jeweils gefolgt von dem oben beschriebenen "Capping" verbliebener freier 5'-OH-Enden, der Oxidation der trivalenten Phosphatgruppen und einer erneuten Abspaltung der DMTr-Schutzgruppen mit TCA.

Die oben beschriebene Synthese entspricht dem dem Fachmann geläufigen Standard der Oligonukleotidsynthese. Im Unterschied zu der geläufigen Standardsynthese werden allerdings die Oligonukleotide derart auf dem festen Träger verankert, dass sie nach der abschließenden vollständigen Abspaltung der Schutzgruppen nicht von dem Träger abgespalten werden, sondern an den Träger gekoppelt bleiben.

Schließlich werden alle Schutzgruppen mit Dichlormethan und Trichloressigsäure abgespalten, der Träger mit Acetonitril gewaschen und getrocknet, sodass im Endeffekt ein Träger mit 4¹² = 16.777.216 verschiedenen Bereichen entsteht, die jeweils eines von allen möglichen über das 3'-Ende gekoppelten 12mer Oligonukleotiden repräsentieren.

### i) Synthese eines Peptidarrays mit Hilfe eines Tintenstrahldruckers

Mit Schutzgruppen, insbesondere mit fmoc Schutzgruppen versehene unterschiedliche einzelne Aminosäuren, insbesondere auch die entsprechenden Anhydride werden zusammen mit Isopropanol oder NMP und Diphenylformamid gelöst. Diese Flüssigkeiten werden in Mehrfarben-Tonerpatronen gefüllt, in einen im wesentlichen handelsüblichen Farbtintenstrahldrucker gesetzt und analog zu der in Beispiel (g) beschriebenen Methode auf Papier gedruckt.

Der beschriebene Tintenstrahldrucker wird dabei vorher analog zu dem bei Beispiel (a) oder (b) beschriebenen Farblaserdrucker derart umgebaut, dass der Träger während repetitiver Druckzyklen relativ zum Druckkopf fixiert bleibt, wobei vorzugsweise eine drehbare Trägerwalze verwendet wird. Wie bei Beispiel (a) oder (b) beschrieben wird ein externes Gerät gebaut, bestehend aus
- einer Heizeinheit, insbesondere durch Infrarotlicht oder durch einen Heißluftfön
- einer drehbaren Walze mit der zeitlich programmierbar verschiedene Flüssigkeiten auf den auf der Trägerwalze fixierten Träger zugeführt werden können
- einer drehbaren Walze mit der zeitlich programmierbar verschiedene Flüssigkeiten von auf dem auf der Trägerwalze fixierten Träger abgeleitet werden können.

Ein geeigneter flacher Träger wird mit Standardmethoden mit freien Aminogruppen derivatisiert. Dabei eignen sich insbesondere Papier oder Kopierfolien, die im wesentlichen aus Polystyrol bestehen. An die freien Aminogruppen des Trägers wird mit Hilfe von dem Fachmann geläufiger Standard fMoc-Peptidsynthese unter wasserfreien Bedingungen zunächst ein geeigneter Spacer, insbesondere von 2-3 Aminosäuren Länge synthetisiert. Optional können diesem Spacer mit Standardmethoden 2 oder 3 weitere Schichten von gekoppelten Aminosäuren hinzugefügt werden, vorzugsweise von Aminosäurengemischen aus 19 oder 20 verschiedenen Aminosäuren (d.h., optional ohne Cystein).

Der Träger kann danach optional mit einer Mischung aus Dichlormethan und Diphenylformamid getränkt werden. Das Dichlormethan wird im Abzug abgedampft, wonach das derivatisierte Papier auf einem relativ zur Druckeinheit des oben beschriebenen umgebauten Farbtintenstrahldruckers beweglichen Trägers fixiert wird.

Anschließend wird der Druckvorgang gestartet, sodass im wesentlichen nach dem Funktionsprinzip eines normalen Farbtintenstrahldruckers, insbesondere 19 oder 20 verschiedene Aminosäurentoner ortsgenau nebeneinander gedruckt werden. Dabei wird der Träger in 19 oder 20 voneinander getrennte, ortsgenau definierte Bereiche unterteilt. Die Kopplung der ortsgenau aufgedruckten aktivierten Aminosäuren findet anschließend bei etwa 65 °C für 5-30 Minuten statt. Während dieses Vorgangs dreht sich die Trägerwalze mit dem darauf fest fixierten derivatisierten Träger gleichmäßig unter einer Zeile von Infrarotlampen, die als Heizeinheit in Beispiel (a) oder (b) beschrieben wurde.

Anschließend wird der nicht umgesetzte Aminosäurentoner mit Hilfe der oben beschriebenen Walzen weggewaschen, die fMoc-Schutzgruppe mit Hilfe von Standardmethoden abgespalten, erneut gewaschen und anschließend der Träger mit Hilfe der oben beschriebenen Heizeinheit getrocknet. Während dieser Zeit bleibt der Träger auf der Trägerwalze fest fixiert, die sich während der ganzen Zeit gleichmäßig dreht.

Anschließend wird der Druckvorgang erneut gestartet, sodass wiederum 19 oder 20 verschiedene Aminosäurentoner ortsgenau neben bzw. übereinander gedruckt werden. Damit wird der Träger diesmal vorzugsweise in 19² bzw. 20² ortsgenau definierte Bereiche unterteilt. Wie gerade beschrieben werden die aktivierten Aminosäuren an den Träger gekoppelt, der nicht umgesetzte Aminosäurentoner weggewaschen und die fmoc Schutzgruppen abgespalten.

Drei weitere analoge Druckvorgänge unterteilen den Träger damit vorzugsweise in 19⁵ bzw. 20⁵ ortsgenau definierte Bereiche. Optional können an die freien Aminotermini dieses Peptidarrays mit Standardmethoden 2 oder 3 weitere Schichten von gekoppelten Aminosäuren hinzugefügt werden, vorzugsweise von Aminosäurengemischen aus 19 oder 20 verschiedenen Aminosäuren (d.h., optional ohne Cystein).

Schließlich werden alle Schutzgruppen, auch die der Seitenketten mit 10% Silan in konzentrierter Trifluoressigsäure abgespalten, der Träger mit DMF und Methanol gewaschen und getrocknet, sodass im Endeffekt ein Träger mit beispielsweise 20⁵ = 3.200.000 verschiedenen Bereichen entsteht, die jeweils eines von allen möglichen natürlich vorkommenden C-terminal gekoppelten Pentapeptiden repräsentieren.

### j) Untersuchung von Blutserum unter Verwendung eines Trägers mit darauf fixierter Peptidbibliothek

Die in Beispiel (g) beschriebene vollständige Pentapeptid-Bibliothek wird mit dem Blutserum eines Patienten gefärbt, wozu zunächst unspezifische Bindungen mit einer geeigneten wäßrigen Lösung, wie zum Beispiel 2 % Milchpulver in PBS abgeblockt werden und das Blutserum im gleichen Puffer verdünnt wird. Danach wird der Träger unter leichtem Schütteln für 60 Minuten mit dem Serum benetzt und anschließend drei Mal gewaschen.

Der Nachweis gebundener menschlicher Antikörper aus dem Blutserum erfolgt mit Standardmethoden. Dazu dienen beispielsweise Ziege-Anti-Mensch-Antikörper oder Antikörperbindeproteine wie Protein G oder Protein A. Diese Nachweisreagentien sind an Enzyme wie Peroxidase oder Phosphatase oder an Farbstoffe oder radioaktive Substanzen wie z.B. Cy5 oder Jod131 gekoppelt.

Die Nachweisreagentien werden in 2 % Milchpulver in PBS verdünnt, der Träger wird damit unter leichtem Schütteln für 60 Minuten benetzt und sodann drei Mal gewaschen. Die Aktivität der Enzyme erzeugt ein farbiges Präzipitat, das mit handelsüblichen Scannern ausgelesen werden kann. Der ortsgenaue Nachweis der gebundenen Radioaktivität oder Fluoreszenz erfolgt mit handelsüblichen Phosphoimagem.

Die Signale werden in insgesamt 10 unterschiedliche Signalstufen unterteilt, die jeweils den unterschiedlichen Pentapeptiden des Peptidarrays zugeordnet werden.

### k) Identifizierung Krankheitsspezifischer Reaktivitäten im Blutserum unter Verwendung eines Trägers mit darauf fixierter Peptidbibliothek

Die in Beispiel (g) beschriebenen Pentapeptidbibliotheken werden wie in Beispiel (j) beschrieben mit dem Blutserum von 50 Patienten mit Magengeschwüren gefärbt. Dabei können mehrere Patientenseren miteinander gemischt werden oder aber pro Serum jeweils ein Array gefärbt werden. Anschließend wird für jedes Peptid des Arrays der Mittelwert der Signalstärke aus mehreren Färbungen ermittelt.

Genau gleich verfahren wird mit 50 Kontrollseren. Auch hierfür wird für jedes Peptid des Arrays der Mittelwert der Signalstärke aus mehreren Färbungen ermittelt.

Durch einen Vergleich der Mittelwerte können einige Peptide identifiziert werden, die von den Patientenseren deutlich stärker gefärbt werden, als von den entsprechenden Kontrollseren. Dies Ergebnis ist schematisch in Fig. 16 dargestellt. Eine Datenbankrecherche mit diesen Peptidsequenzen ergibt dabei eine Verwandschaft dieser Peptide mit den Genprodukten des Bakteriums Helicobacter pylori.

### l) Untersuchung von Patienten-DNA unter Verwendung einer auf einem Träger fixierten 12mer Oligonukleotidbibliothek

Der unter Beispiel (h) beschriebene Träger mit einer darauf fixierten vollständigen Oligonukleotid-Bibliothek wird mit Patienten-DNA gefärbt. Dabei werden dem Fachmann bekannte Standardmethoden verwendet. Unspezifische Bindungen werden z.B. mit DNA aus Heringsspermien abgesättigt.

Dem Patienten wird eine Tumor-Gewebsprobe und gleichzeitig eine Probe mit gesundem Gewebe entnommen und die darin enthaltene genomische DNA mit Hilfe eines oder mehrerer Paare von Tumorgen-spezifischen Primern (spezifisch z.B. für die Gene von p53, p16, ras, c-myc, n-myc) in einer Polymerase-Kettenreaktion vervielfältigt. Dabei wird die Tumorprobe beispielsweise mit Fluorescein-12-dUTP markiert, während die Normalprobe beispielsweise mit Tetramethyl-rhodamin-5-dUTP markiert wird. Die Proben werden miteinander, gemischt und auf den Träger hybridisiert.

Der ortsgenaue Nachweis der gebundenen Fluoreszenzen (oder Radioaktivität) erfolgt mit handelsüblichen Phosphoimagem wie auch in Beispiel (j) beschrieben. Dabei wird beispielsweise das Verhältnis von grüner zu roter Fluoreszenz bzw. die resultierende Mischfarbe bestimmt. Die Signale werden jeweils den unterschiedlichen 12er Oligonukleotiden des Arrays zugeordnet

Auf diese Weise können Punktmutationen in Genen, die für die Prognose von Tumorerkrankungen wichtig sind, diagnostiziert werden. Im Unterschied zu den auf dem Markt befindlichen Systemen können mit einer vollständigen 12mer Oligonukleotidbibliothek jedoch viele Gene gleichzeitig analysiert werden.

In einem alternativen Ansatz dient die dem Patienten entnommene DNA als Template für die Vervielfältigung mit sogenannten Alu-Primern, die an die Ränder von sehr häufig im Genom vorkommenden repetitiven Alu-Sequenzen hybridisieren und die zwischen 2 Alusequenzen liegende nicht-repititive DNA vervielfältigen. Wieder wird die Tumorprobe beispielsweise mit Fluorescein-12-UTP markiert, während Tetramethylrhodamin-5-dUTP in die Normalprobe eingebaut wird. Die Proben werden wie oben beschrieben miteinander gemischt und anschließend auf den Träger hybridisiert.

Das Auslesen der Fluoreszenzsignale erfolgt anschließend wie oben beschrieben. Auf diese Weise wird ein sehr großer Teil des Genoms auf Unterschiede zwischen normalem und Tumorgewebe abgerastert, wodurch neue diagnostische Marker entdeckt werden können, die wichtige Informationen für die Tumorprogression liefern.

### Bezugszeichenliste

- 1: Träger
- 2: Substanzen
- 3: Matrix
- 4: erstes Lösungsmittel
- 5: Transporteinheit
- 6: Bewegung in Richtung Träger (1)
- 7: fester oder Gel-artiger Aggregatszustand
- 8: geänderte physikalische Umgebung
- 9: beweglich gemachte Substanzen
- 10: Bewegung in Richtung Trägeroberfläche (1)
- 11: kovalent an den Träger (1) gekoppelte Substanzen (2)
- 12: zweites Lösungsmittel
- 13: flüssiger Aggregatszustand
- 14: Schutzgruppe
- 15: mit Licht abspaltbare Schutzgruppe
- 16: elektromagnetische Wellen, Licht
- 17: Licht empfindliche Schutzschicht
- 18: orstgenau definierter Bereich auf einem Array bzw. Träger (1)
- 19: Tonerpartikel, Transporteinheit,
- 20: Tonerreservoir
- 21: Magnetwalze
- 22: durch Laserlicht beschreibbare Walze
- 23: geladene Tonerpartikel springen auf die beschreibbare Walze (22)
- 24: Tonerpartikel auf dem Träger (1)
- 25: heiße Walze
- 26: eingeschmolzene, bzw. beweglich gemachte Tonerpartikel
- 27: erste aufs Papier aufgedruckte Farbe
- 28: zweite aufs Papier aufgedruckte Farbe
- 29: dritte aufs Papier aufgedruckte Farbe
- 30: vierte aufs Papier aufgedruckte Farbe
- 31: Tonerreservoir mit Tonerpartikeln (19) der ersten Farbe
- 32: Tonerreservoir mit Tonerpartikeln ( 19) der zweiten Farbe
- 33: Tonerreservoir mit Tonerpartikeln (19) der dritten Farbe
- 34: Tonerreservoir mit Tonerpartikeln (19) der vierten Farbe
- 35: Trägerwalze oder Transfereinheit eines Farblaserdruckers
- 36: Zufuhr von Kopplungsreagentien, Waschlösungen oder gasförmigen Substanzen
- 37: Scannereinheit
- 38: ein auf den Träger (1) aufgebrachtes Muster
- 39: Vergleich des mit der Scannereinheit (37) gemessenen Musters mit demselben bereits gespeicherten Muster
- 40: elektronische Verschiebung des im Druckerspeicher vorliegenden Bildes um die Abweichung vom Sollwert
- 41: unbenutzt
- 42: Peptide, die sowohl von Patientenseren, wie von Kontrollseren erkannt werden
- 43: Peptide, die spezifisch von Patientenseren erkannt werden
- 44: D-Peptide des Arrays, die das Enzym binden
- 45: D-Peptide des Arrays, die das Enzym binden und gleichzeitig inaktivieren
- 46: magnetischer Anteil
- 47: aufschmelzbarer Kunstoffanteil
- 48: Chromophoren
- 49: Monomer für die kombinatorische Synthese Substanz (2)
- 50: Lösungsmittel im Aggregarszustand fest

## Patentansprüche

1. Verfahren zum Aufbringen von Substanzen auf einen Träger, insbesondere von Monomeren für die kombinatorische Synthese von Molekülbibliotheken, ***dadurch gekennzeichnet*,**
- ***dass*** die Substanzen (2) zunächst in einer Matrix (3) bestehend aus mindestens einem ersten Lösungsmittel (4) eingebettet sind, das bei einer Temperatur von < 90°C, vorzugsweise bei einer Temperatur von < 50°C im festen Aggregatszustand (7) vorliegt,
- ***dass*** die genannten Substanzen (2) eingebettet in der genannten Matrix (3) bestehend aus mindestens einem ersten Lösungsmittel (4) Transporteinheiten (5) bilden, die als Einheiten bewegt (6) werden,
- ***dass*** die genannten Transporteinheiten (5) anschließend bei einer Temperatur von < 90°C, vorzugsweise bei einer Temperatur von < 50°C im festen Aggregatszustand (7) auf den Träger (1) aufgebracht werden (6).
- ***dass*** die genannten Transporteinheiten (5) nach dem Aufbringen auf einen Träger (1) in einem festen oder Gel-artigen Aggregatszustand (7) verbleiben,
- ***dass*** anschließend die genannten in dem genannten ersten Lösungsmittel gelösten Substanzen (2), die sich auf dem Träger befinden (1), durch Veränderung der physikalischen Umgebung (8) beweglich gemacht werden (9), insbesondere innerhalb des genannten ersten Lösungsmittels (4),
- ***dass*** die so beweglich gemachten Substanzen (2, 9) durch einen physikalischen Prozeß in die Nähe der Trägeroberfläche gelangen (10),
- ***dass*** die so beweglich gemachten Substanzen (2, 9) kovalent an auf dem Träger ( 1 ) befindliche Moleküle koppeln, oder mit diesen eine chemische Reaktion eingehen oder diese katalysieren,
- ***dass*** die so beweglich gemachten, an den Träger gekoppelten Substanzen (11) viele unterschiedliche Substanzen (2) sind oder ergeben,
- ***dass*** wiederholbar hintereinander mehr als eine Schicht der genannten Substanzen (2) ortsgenau auf den Träger (1) aufgebracht wird, jeweils gefolgt von dem kovalenten Koppeln der Substanzen an den Träger (11) und dem Wegwaschen nicht gekoppelter Substanzen.

2. Verfahren nach Anspruch 1, ***dadurch gekennzeichnet, dass*** die genannte Substanz (2) bei einer Temperatur von < 90°C, vorzugsweise bei einer Temperatur von < 50°C in Partikeln einer Größe von 0,2 µm bis 200 µm, vorzugsweise einer Größe von 2 µm bis 40 µm immobilisiert vorliegt.

3. Verfahren nach Anspruch 1 oder 2, ***dadurch gekennzeichnet, dass*** der Träger ( 1 ) bis zum Start der Kopplungsreaktion (10, 11) der genannten Monomere an den Träger (1) bei einer mindestens um 10°C tieferen Temperatur gehalten wird verglichen mit der genannten Transporteinheit (7, 13).

4. Verfahren nach einem der Ansprüche 1 bis 3, ***dadurch gekennzeichnet, das*s** der ortsgenaue Transfer der Substanzen mit Hilfe eines entsprechend modifizierten Druckverfahrens, insbesondere mit Hilfe eines Laserdruckers oder eines Laserkopierers erfolgt.

5. Verfahren nach einem der Ansprüche 1 bis 4, ***dadurch gekennzeichnet, dass*** der ortsgenaue Transfer der Substanzen mit Hilfe einer Anzahl von gezielt ansteuerbaren Lichtquellen, insbesondere mit Hilfe eines Arrays von Leuchtdioden oder Mikrolasern, erfolgt.

6. Verfahren nach einem der Ansprüche 1 bis 5, ***dadurch gekennzeichnet, dass*** die auf den Träger aufzubringenden Partikel über den Träger zerstäubt werden.

7. Verfahren nach einem der Ansprüche 1 bis 6, ***dadurch gekennzeichnet, dass*** die Partikel auf dem Träger gekühlt und tiefgefroren werden.

8. Verfahren nach einem der Ansprüche 1 bis 7, ***dadurch gekennzeichnet, dass*** die Partikel wenigstens ein Element der folgenden Gruppe enthalten oder an solche Partikel binden, die ein Element aus der folgenden Gruppe enthalten: magnetische Bestandteile, Diphenylformarnid, Vorstufen für von für eine kombinatorische Synthese geeigneten Monomeren, Dimeren oder Trimeren, insbesondere Vorstufen D- oder L- Aminosäuren, von Nukleosiden oder von derivatisierten Nukleosiden oder von ihren Spiegelbildern oder ihren Derivaten oder Polystyrol oder Zellulose, insbesondere Polystyrol oder Zellulose an das eine oder mehrere Schichten von Monomeren gekoppelt sind.

9. Verfahren nach einem der Ansprüche 1 bis 8, ***dadurch gekennzeichnet, dass*** nach einem ersten Zyklus von Kopplungsreaktionen Schutzgruppen mit Standardmethoden derart abgespalten werden, dass vorzugweise freie Aminogruppen oder Hydroxylgruppen entstehen, an die Vorstufen von Monomeren, Dimeren oder Trimeren koppeln können.

10. Verfahren nach einem der Ansprüche 1 bis 9, ***dadurch gekennzeichnet, dass*** als Träger Polystyrolfolien, Papier, CDs, MODs, DVDs oder FMDs, verwendet werden.

11. Verfahren nach einem der Ansprüche 1 bis 10, ***dadurch gekennzeiehnet, dass*** die immobilisierten, Substanzen (Tonerpartikel) durch das Anlegen einer elektrischen Spannung bewegt werden.

12. Verfahren zum Aufbringen von Substanzen auf einen Träger, nach einem der Ansprüche 1 bis 11, ***dadurch gekennzeichnet dass*** *elekt* romagnetische Wellen, insbesondere Laserlicht, wiederholbar, ortsgenau auf ausgewählte Bereiche des Trägers eingestrahlt werden, dass die ausgewählten Bereiche des Trägers mit unterschiedlichen Molekülen oder mit unterschiedlichen Aggregaten an diese Moleküle beladen sind oder beladen werden, dass die unterschiedlichen Moleküle oder Aggregate an diesen Molekülen in Wechselwirkung treten, dass die sich auf den ausgewählten Bereichen befindenden unterschiedlichen Moleküle oder die Aggregate an diesen Molekülen oder die anderen Moleküle mit den eingestrahlten elektromagnetischen Wellen wechselwirken und dass sie durch die Wechselwirkung der eingestrahlten elektromagnetischen Wellen mit den Molekülen oder mit Aggregaten an diesen Molekülen oder mit anderen Molekülen lokale, physikalische oder chemische Prozesse in Gang setzen.

13. Vorrichtung zum Aufbringen von Molekülen oder Substanzen nach einem der vorhergehenden Verfahren auf eine im wesentlichen ebene Oberfläche eines Trägers, umfassend Mittel zur drehbaren Halterung des Trägers, um eine zu der genannten Oberfläche des Trägers im wesentlichen senkrechte Drehachse, Mittel zum Aufbringen verschiedener Flüssigkeiten auf die Oberfläche des Trägers im Bereich der Drehachse und wenigstens einem relativ zum Träger verfahrbaren Laser zum Bestrahlen ausgewählter Bereiche des Trägers mit Laserlicht.

14. Vorrichtung zum Aufbringen von Molekülen oder Substanzen nach einem der vorhergehenden Verfahren auf eine im wesentlichen ebene Oberfläche eines Trägers, umfassend düsenartige Mittel zum Aufbringen kleinster Mengen auf dem Träger zu verankernder Moleküle. Mittel zum Verfahren der Mittel zum Aufbringen der Moleküle und des Trägers relativ zueinander und wenigstens einem Laser zur Bestrahlung ausgewählter Bereiche des Trägers mit Laserlicht.

15. Vorrichtung zum Aufbringen von Molekülen oder Substanzen nach einem der vorhergehenden Verfahren auf eine im wesentlichen ebene Oberfläche eines Trägers, umfassend einen Behälter für die die aufzubringenden Moleküle enthaltenden Partikel, einen Laser und Mittel zum Bewegen des Trägers und des Lasers relativ zueinander.

16. Vorrichtung nach Anspruch 15, ***dadurch gekennzeichnet, dass*** es sich bei der Vorrichtung um einen modifizierten, ansonsten aber im wesentlichen handelsüblichen Laserdrucker oder Laserkopierer handelt, bei welchem die Tonerpartikel durch die aufzubringenden Moleküle enthaltende Partikel ersetzt sind.

17. Vorrichtung nach Anspruch 16, ***dadurch gekennzeichnet, dass*** eine Rückkoppelungseinrichtung die Trägerwalze oder eine Trägereinheit (des Laserdruckers) in Bezug auf die (Laser-) beschreibbare Walze justiert.

18. Vorrichtung nach Anspruch 17, ***dadurch gekennzeichnet, dass*** durch die Ruckkoppetungseinrichtung die räumliche Beziehung zeitlich nacheinander wiederholt hergestellt wird, wobei diese räumliche Wiederherstelibarkeit auch systemübergreifend, dass heißt zwischen verschiedenen Laserdruckern funktioniert.

19. Vorrichtung nach Anspruch 17 oder 18, ***dadurch gekennzeichnet, dass*** die Rückkopplungseinrichtung ein Raster von Positionsmarkierungen verwendet, die auf dem Träger, der Trägerwalze oder der Transfereinheit aufgebracht sind.

20. Vorrichtung nach Anspruch 19, ***dadurch gekennzeichnet, dass*** die Rückkopplungseinrichtung durch elektronische Verschiebung der Bildpunkte im Druckerspeicher die Abweichung der Positionsmarkierungen in Bezug auf ein vorher gespeichertes Raster korrigiert.

21. Vorrichtung nach einem der Ansprüche 17 bis 20, ***dadurch gekennzeichnet, dass*** der Rückkopplungsmechansismus durch eine exakte mechanische Kopplung realisiert ist.

22. Vorrichtung nach einem der Ansprüche 17 bis 21, ***dadurch gekennzeichnet, dass*** die Rückkopplungseinrichtung sowohl mechanisch wie auch elektronisch realisiert ist.

## Claims

1. A method for applying substances, more specifically monomers for combinatorial synthesis of molecular libraries, onto a carrier, ***characterized in that***
- ***that*** the substances (2) are at first embedded in a matrix (3) consisting of at least one first solvent (4) that is present in a solid state of aggregation at a temperature of < 90°C, preferably at a temperature of < 50°C,
- ***that*** said substances (2) embedded in said matrix (3) consisting of at least one first solvent (4) form transport units (5) that are moved (6) as units,
- ***that*** said transport units (5) are then applied (6) on the carrier (1) in a solid state of aggregation (7) at a temperature of < 90°C, preferably at a temperature of < 50°C,
- ***that*** said transport units (5) remain in a solid or viscous state of aggregation (7) after having been applied on the carrier (1),
- ***that*** said substances (2) dissolved in said first solvent and located on the carrier (1) are made mobile (9) more specifically inside said first solvent (4) by changing the physical environment (8),
- ***that*** the substances (2, 9) thus made mobile come close (10) to the surface of the carrier by a physical process,
- ***that*** the substances (2,9) thus made mobile are covalently coupled to molecules located on the carrier (1) or react chemically with these or catalyze such a reaction,
- ***that*** the substances (11) thus made mobile and coupled to the carrier are or yield many different substances,
- more than one layer of said substances (2) are repeatably and in terms of location accurately applied one after the other on the carrier (1), respectively followed by covalently coupling the substances to the carrier (11) and by washing the non coupled substances away.

2. The method according to claim 1, ***characterized in that*** said substance (2) is immobilized in the form of particles having a size of 0.2 µm to 200 µm, preferably a size of 2 µm to 40 µm at a temperature of < 90°C, preferably at a temperature of < 50°C.

3. The method according to claim 1 or 2, ***characterized in that*** the carrier (1) is held at a temperature at least 10°C lower as compared to said transport unit (7, 13) until the reaction (10, 11) of coupling said monomers to the carrier (1) is started.

4. The method according to one of the claims 1 to 3, ***characterized in that*** the substances are in terms of location accurately transferred using an accordingly modified printing method, more specifically a laser printer or a laser photocopier.

5. The method according to one of the claims 1 to 4, ***characterized in that*** the substances are accurately transferred in terms of location by means of selectively activatable light sources, more specifically by means of an array of light emitting diodes or micro lasers.

6. The method according to one of the claims 1 to 5, ***characterized in that*** the particles that are to be applied on the carrier are atomized over the carrier.

7. The method according to one of the claims 1 to 6, ***characterized in that*** the particles are cooled and deep-frozen on the carrier.

8. The method according to one of the claims 1 to 7, ***characterized in that*** the particles contain at least one element of the following group or are bound to such particles that contain an element of the following group: magnetic components, diphenylformamide, precursors of monomers, dimers or trimers that are suited for a combinatorial synthesis, more specifically precursors of D or L amino acids, of nucleosides or of derived nucleosides or of their mirror images or their derivatives or polystyrene or cellulose, more specifically polystyrene or cellulose on which are coupled one or plurality layers of monomers.

9. The method according to one of the claims 1 to 8, ***characterized in that*** after a first cycle of coupling reactions protecting groups are separated using standard methods in such a manner that preferably free amino groups or hydroxyl groups are generated on which the precursors of monomers, dimers or trimers can be coupled.

10. The method according to one of the claims 1 to 9, ***characterized in that*** polystyrene film, paper, CDs, MODs, DVDs or FMDs can be used as the carrier.

11. The method according to one of the claims 1 to 10, ***characterized in that*** the immobilized substances (toner particles) are moved by applying a electric voltage.

12. A method for applying substances on a carrier according to one of the claims 1 to 11, ***characterized in that*** electromagnetic waves, more specifically laser light, repeatably and in terms of location accurately irradiate selected areas of the carrier, that the selected areas of the carrier are loaded or are being loaded with different molecules or with different aggregates on these molecules, that the different molecules or aggregates on these molecules start to interact with each other, that the different molecules or the aggregates on these molecules or the other molecules located on the selected areas interact with the irradiating electromagnetic waves and that they initiate physical or chemical processes through the interaction of the irradiating electromagnetic waves with the molecules or with aggregates on these molecules or with other molecules.

13. A device for applying molecules or substances according to one of the aforementioned methods on a substantially level surface of a carrier, said device including means for rotatably holding the carrier around a rotational axis that is substantially vertical relative to said surface of the carrier, means for applying different liquids on the surface of the carrier in the area of the rotational axis and at least one laser that is movable relative to the carrier for irradiating selected areas of the carrier with laser light.

14. A device for applying molecules or substances according to one of the aforementioned methods on a substantially level surface of a carrier, including nozzle type means for applying minute quantities of molecules that have to be anchored on the carrier, means for displacing the means for applying the molecules and the carrier relative to each other and at least one laser for irradiating selected areas of the carrier with laser light.

15. A device for applying molecules or substances according to one of the aforementioned methods on a substantially level surface of a carrier, including a container for the particles containing the molecules that are to be applied, a laser and means for moving the carrier and the laser relative to each other.

16. The device according to claim 15, ***characterized in that*** the device is a modified laser printer or laser photocopier but otherwise is substantially usual in commerce and in which the toner particles are replaced by the particles containing the molecules to be applied.

17. The device according to claim 16, ***characterized in that*** a feedback apparatus adjusts the carrier cylinder or the carrier unit (of the laser printer) relative to the (laser-) writable roll.

18. The device according to claim 17, ***characterized in that*** the spatial relationship is successively and repeatedly recreated by means of the feedback apparatus, this spatial recreatability being also interoperable, which means that it functions using different laser printers.

19. The device according to claim 17 or 18, ***characterized in that*** the feedback apparatus uses a grid of positional markers that are applied on the carrier, the carrier cylinder or the transfer unit.

20. The device according to claim 19, ***characterized in that*** the feedback apparatus corrects the deviation of the positional markers relative to a previously saved grid by electronically displacing the picture elements in the printer memory.

21. The device according to one of the claims 17 to 20, ***characterized in that*** the feedback mechanism is realised by a precise mechanical coupling.

22. The device according to one of the claims 17 to 21, ***characterized in that*** the feedback mechanism is realised mechanically as well as electronically.

## Revendications

1. Procédé d'application de substances sur un support, notamment de monomères pour la synthèse combinatoire de bibliothèques de molécules, ***caractérisé en ce***
- ***que*** les substances (2) sont tout d'abord noyées dans une matrice (3) constituée d'au moins un premier solvant (4) présent sous forme d'agrégat solide (7) à une température < 90 °C, de préférence à une température <50 °C,
- ***que*** lesdites substances (2) noyées dans ladite matrice (3) constituée d'au moins un premier solvant (4) forment des unités de transport (5) qui sont déplacées (6) en tant qu'unités,
- ***que*** lesdites unités de transport (5) sont ensuite appliquées (6) sur le support (1) sous forme d'agrégat solide (7) à une température < 90 °C, de préférence à une température <50 °C,
- ***que*** lesdites unités de transport (5), une fois appliquées sur un support (1), conservent la forme d'agrégat (7) solide ou visqueux,
- ***que*** lesdites substances (2) dissoutes dans ledit premier solvant et se trouvant sur le support (1) sont rendues mobiles (9) par modification de l'environnement physique (8), notamment au sein dudit premier solvant (4),
- ***que*** les substances (2, 9) rendues mobiles de cette façon parviennent (10) par un processus physique à proximité de la surface du support,
- ***que*** les substances (2, 9) rendues mobiles de cette façon sont couplées de façon covalente à des molécules se trouvant sur le support (1) ou réagissent chimiquement avec celles-ci ou catalysent cette réaction,
- ***que*** les substances (11) rendues mobiles de cette façon et couplées au support sont ou donnent lieu à beaucoup de substances différentes (2),
- ***que*** plus d'une couche desdites substances (2) est appliquée sur un endroit précis du support (1), ce processus pouvant être répété plusieurs fois de suite et étant suivi du couplage covalent des substances au support (11) et du lavage des substances non couplées.

2. Procédé selon la revendication 1, ***caractérisé en ce que,*** à une température < 90 °C, de préférence à une température <50 °C, ladite substance (2) est immobilisée sous forme de particules dont la taille est comprise entre 0,2 µm et 200 µm, de préférence entre 2 µm et 40 µm.

3. Procédé selon la revendication 1 ou 2, ***caractérisé en ce que,*** jusqu'à ce que la réaction de couplage (10, 11) desdits monomères au support (1) soit initiée, le support (1) est maintenu à une température inférieure d'au moins 10 °C à celle de ladite unité de transport (7, 13).

4. Procédé selon l'une quelconque des revendications 1 à 3, ***caractérisé en ce que*** les substances sont transférées à un endroit précis à l'aide d'un procédé d'impression modifié en conséquence, notamment à l'aide d'une imprimante laser ou d'une photocopieuse laser.

5. Procédé selon l'une quelconque des revendications 1 à 4, ***caractérisé en ce que*** les substances sont transférées à un endroit précis à l'aide d'un nombre de sources lumineuses aptes à être activées sélectivement, notamment à l'aide d'un réseau de diodes luminescentes ou de microlasers.

6. Procédé selon l'une quelconque des revendications 1 à 5, ***caractérisé en ce que*** les particules destinées à être appliquées sur le support sont atomisées au-dessus du support.

7. Procédé selon l'une quelconque des revendications 1 à 6, ***caractérisé en ce que*** les particules sont refroidies et congelées sur le support.

8. Procédé selon l'une quelconque des revendications 1 à 7, ***caractérisé en ce que*** les particules contiennent au moins un élément du groupe suivant ou se lient à des particules contenant un élément du groupe suivant : des composants magnétiques, du diphénylformamide, des précurseurs de monomères, dimères ou trimères se prêtant à une synthèse combinatoire, notamment des précurseurs des acides aminés D ou L, de nucléosides ou de nucléosides dérivés ou de leurs symétriques ou de leurs dérivés ou du polystyrène ou de la cellulose, notamment du polystyrène ou de la cellulose à laquelle sont couplées une ou plusieurs couches de monomères.

9. Procédé selon l'une quelconque des revendications 1 à 8, ***caractérisé en ce qu*'**après un premier cycle de réactions de couplage des groupes protecteurs sont séparés au moyen de procédés courants de manière à obtenir de préférence des groupes amino ou hydroxy libres auxquels peuvent se coupler des précurseurs de monomères, dimères ou trimères.

10. Procédé selon l'une quelconque des revendications 1 à 9, ***caractérisé en ce que*** l'on utilise comme support des feuilles de polystyrène, des supports papier, CD, DMO, DVD ou FMD.

11. Procédé selon l'une quelconque des revendications 1 à 10, ***caractérisé en ce que*** les substances immobilisées (particules de toner) sont mises en mouvement par l'application d'une tension électrique.

12. Procédé d'application de substances sur un support selon l'une quelconque des revendications 1 à 11, ***caractérisé en ce que*** des ondes électromagnétiques, notamment de la lumière laser, irradient de manière répétitive un endroit précis de régions sélectionnées du support, que les régions sélectionnées du support sont chargées de molécules différentes ou d'agrégats différents sur ces molécules ou vont l'être, que les différentes molécules ou les différents agrégats sur ces molécules commencent à interagir, que les différentes molécules ou les agrégats sur ces molécules ou les autres molécules se trouvant dans les régions sélectionnées interagissent avec les ondes électromagnétiques qui les irradient et que par l'interaction des ondes électromagnétiques irradiant ces régions avec les molécules ou avec les agrégats sur ces molécules ou avec d'autres molécules, celles-ci initient des processus locaux physiques ou chimiques.

13. Dispositif destiné à appliquer des molécules ou des substances selon l'un des procédés précédents sur une surface sensiblement plane d'un support, du type comprenant des moyens de retenue du support monté mobile en rotation autour d'un axe de rotation sensiblement perpendiculaire à ladite surface du support, des moyens d'application de différents liquides sur la surface du support dans la région de l'axe de rotation et au moins un laser mobile en translation par rapport au support et destiné à irradier de lumière laser des régions sélectionnées du support.

14. Dispositif destiné à appliquer des molécules ou des substances selon l'un des procédés précédents sur la surface sensiblement plane d'un support, du type comprenant des moyens du type buse destinés à appliquer sur le support des quantités infimes de molécules destinées à y être fixées, des moyens destinés à déplacer les moyens d'application des molécules et le support les uns par rapport à l'autre et au moins un laser destiné à irradier de lumière laser des régions sélectionnées du support.

15. Dispositif destiné à appliquer des molécules ou des substances selon l'un des procédés précédents sur la surface sensiblement plane d'un support, du type comprenant un récipient pour les particules contenant les molécules destinées à être appliquées, un laser et des moyens destinés à déplacer le support et le laser l'un par rapport à l'autre.

16. Dispositif selon la revendication 15, ***caractérisé en ce que*** le dispositif est une imprimante laser ou une photocopieuse laser modifiée mais par ailleurs sensiblement disponible sur le marché dans laquelle on a remplacé les particules de toner par des particules contenant les molécules destinées à être appliquées.

17. Dispositif selon la revendication 16, ***caractérisé en ce que*** le système de rétroaction ajuste le support rouleau ou une unité support (de l'imprimante laser) par rapport au rouleau gravable (au laser).

18. Dispositif selon la revendication 17, ***caractérisé en ce que****,* grâce au système de rétroaction, la relation spatiale est rétablie plusieurs fois de suite, cette aptitude au rétablissement fonctionnant également entre différents systèmes, c'est-à-dire entre différentes imprimantes laser.

19. Dispositif selon la revendication 17 ou 18, ***caractérisé en ce que*** le système de rétroaction utilise une grille de marquage de positionnement, ce marquage étant apposé sur le support, le support rouleau ou l'unité de transfert.

20. Dispositif selon la revendication 19, ***caractérisé en ce que*** le système de rétroaction corrige l'écart entre le marquage de positionnement et une grille mise en mémoire au préalable par déplacement électronique des points image dans la mémoire de l'imprimante.

21. Dispositif selon l'une quelconque des revendications 17 à 20, ***caractérisé en ce que*** le mécanisme de rétroaction est réalisé par un couplage mécanique précis.

22. Dispositif selon l'une quelconque des revendications 17 à 21, ***caractérisé en ce que*** le système de rétroaction est réalisé tant mécaniquement qu'électroniquement.
